(19) Europäisches Patentamt
European Patent Office
Office européen des brevets

(11) **EP 2 285 913 B1**

(12) **EUROPÄISCHE PATENTSCHRIFT**

(45) Veröffentlichungstag und Bekanntmachung des Hinweises auf die Patenterteilung:
**17.09.2014 Patentblatt 2014/38**

(21) Anmeldenummer: **09765521.1**

(22) Anmeldetag: **28.05.2009**

(51) Int Cl.:
*C09C 1/62* (2006.01)     *C09C 1/66* (2006.01)
*C09D 5/36* (2006.01)     *C09D 7/12* (2006.01)
*C09D 11/02* (2014.01)     *A61K 8/11* (2006.01)
*A61Q 1/02* (2006.01)     *A61K 8/19* (2006.01)
*B82Y 5/00* (2011.01)

(86) Internationale Anmeldenummer:
**PCT/EP2009/003793**

(87) Internationale Veröffentlichungsnummer:
**WO 2009/152941 (23.12.2009 Gazette 2009/52)**

(54) **PLÄTTCHENFÖRMIGE KUPFERHALTIGE METALLEFFEKTPIGMENTE, VERFAHREN ZU DEREN HERSTELLUNG UND VERWENDUNG DERSELBEN**

PLATELET-SHAPED, COPPER-CONTAINING, METALLIC EFFECT PIGMENTS, PROCESS FOR PREPARING THEM AND USE THEREOF

PIGMENTS À EFFET MÉTALLIQUE CONTENANT DU CUIVRE, EN FORME DE PAILLETTES, PROCÉDÉS DE FABRICATION ET UTILISATION DE CES PIGMENTS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **28.05.2008 EP 08009699**

(43) Veröffentlichungstag der Anmeldung:
**23.02.2011 Patentblatt 2011/08**

(73) Patentinhaber: **Eckart GmbH**
**91235 Hartenstein (DE)**

(72) Erfinder:
• **PROELSS, Dieter**
**91126 Schwabach (DE)**
• **DEINZER, Ralf**
**91284 Neuhaus (DE)**
• **HERZING, Wolfgang**
**91233 Neunkirchen (DE)**

• **KROELL, Andreas**
**91235 Velden (DE)**
• **TRUMMER, Stefan**
**90480 Nürnberg (DE)**
• **BECKER, Michael**
**91207 Lauf (DE)**
• **WCZASEK, Karin**
**90419 Nürnberg (DE)**

(74) Vertreter: **Louis Pöhlau Lohrentz**
**Patentanwälte**
**Postfach 30 55**
**90014 Nürnberg (DE)**

(56) Entgegenhaltungen:
**EP-A- 1 553 144     WO-A-2004/026972**
**WO-A-2006/066825     DE-A1- 10 315 775**
**DE-A1-102006 051 893     GB-A- 1 264 584**
**US-A- 4 884 754     US-A1- 2002 050 186**
**US-B1- 6 398 861**

EP 2 285 913 B1

**Beschreibung**

Die Erfindung betrifft plättchenförmige kupferhaltige Metalleffektpigmente, deren Herstellung und Verwendung sowie eine diese Pigmente enthaltende Beschichtungszusammensetzung.

Kupferhaltige Metalleffektpigmente, umfassend Kupferpigmente oder aus einer Kupfer-Zink-Legierung hergestellte Messingpigmente, auch als Goldbronzepigmente bezeichnet, werden u. a. auch in der grafischen Industrie, beispielsweise in Druckfarben, eingesetzt.

Die bisher zur Pigmentierung von Flexo- und Tiefdruckfarben eingesetzten, durch Vermahlung (konventionell überwiegend im Trockenmahlprozess) aus Kupfer- oder Messinggrieß hergestellten Metalleffektpigmente, wie beispielsweise Goldbronzepigment-Dispersionen "ROTOVARIO" oder stabilisiertes leafing Goldbronzepigment-Pulver "ROTOFLEX" bzw. stabilisierte leafing Goldbronzepigment-Pellets "ROTOSAFE" der Fa. Eckart GmbH, D-90763 Fürth, Deutschland, sind aufgrund ihrer leafing-Eigenschaften für die Pigmentierung von Folienkonterapplikationen mit spiegelartigem Effekt nur bedingt geeignet.

Auch der Einsatz von durch PVD-Verfahren hergestellten Messingpigmenten in Druckfarben ist insofern problematisch, da eine homogene Metallisierung der zwei Metalle (Kupfer und Zink) zur Erzielung eines gleichmäßigen Farbtones mit stark unterschiedlichen Verdampfungstemperaturen im Hochvakuum verfahrenstechnisch extrem schwierig ist. Zudem weisen die in der Herstellung relativ teuren PVD-Messingpigmente - im Gegensatz zu durch konventionelle Vermahlung hergestellten Messingpigmenten - weniger kompakte Schichten mit Dichten unterhalb der jeweiligen Materialdichten auf und die gewünschten Goldfarbtöne (vor allem Reichgold) lassen sich nicht bei den gewünschten niedrigen Schichtdicken realisieren.

Die EP 1 529 084 B1 beschreibt Goldbronzepigmente, die durch PVD-Verfahren hergestellt werden können. Diese Pigmente sind aufgrund des aufwändigen Verfahrens sehr teuer. Zudem neigen diese Pigmente zu einer teilweisen Phasenseparation der Legierungsbestandteile, was ebenfalls unerwünschte Farbtonverschiebungen und nicht ausreichende Farbtonstabilitäten mit sich bringt.

Auch sind bislang noch keine mittels PVD-Verfahren hergestellten kupferhaltigen Effektpigmente kommerziell erhältlich.

Die deshalb i. d. R. zur Pigmentierung von Folienkonterapplikationen mit spiegelartigem Effekt eingesetzten PVD-Aluminiumpigmente, wie beispielsweise die Handelsprodukte "METALURE" der Eckart GmbH weisen zwar eine homogene Oberflächenbeschaffenheit mit einem perfekten non-leafing-Verhalten auf, müssen aber mit gelblichen Tonerpigmenten farbig eingefärbt werden. Derartige Druckfarben werden beispielsweise als lösemittelbasierende Tief- und Flexodruckfarben unter der Produktbezeichnung "ULTRASTAR" von der Fa. Eckart kommerziell gehandelt. Aufgrund der aufwändigen Herstellungsweise sind PVD-Metalleffektpigmente generell weitaus teurer als mittels Vermahlung, auf konventionelle Weise hergestellte Metalleffektpigmente.

Mit diesen Druckfarben kann in der sogenannten Konterapplikation, bei der eine transparente Folie mit der Druckfarbe bedruckt wird, ein nahezu perfekter Metallspiegel erzeugt werden. Dabei wird jedoch der Spiegel nur sichtbar, wenn man die Applikation von der Folienseite her betrachtet. Aufgrund des beigemischten gelblichen Toners erscheint der Metallspiegel in goldenen Farbtönen. Der Silberglanz der Aluminiumpigmente mischt sich entsprechend mit der Eigenfarbe der Farbstoffe.

Diese Druckfarben weisen den Nachteil auf, dass sie nicht sehr farbintensiv sind. Die PVD-Aluminiumpigmente neigen dazu, sich in Nähe der Folie, d.h. eher im unteren Bereich des Druckfilmes anzusammeln. Somit ist jedoch zwischen Aluminiumpigmenten und Folie entsprechend wenig Farbpigment vorhanden, was die Farbkraft mindert. Erhöht man zum Ausgleich die Farbpigmentkonzentration so müssen korrespondierende Glanzverluste in Kauf genommen werden.

Weiterhin hat die Druckfarbe Nachteile, wenn sie auf saugende Untergründe wie beispielsweise Papier aufgetragen wird, da es zu einer Trennung zwischen Metallpigment und Farbpigment kommen kann.

Zur Herstellung von durch Vermahlung aus Messinggrieß hergestellten, konventionellen Messingpigmenten werden als Ausgangsmaterial für den Mahlprozess hochreines, elektrolytisch gewonnenes Kupfer und Zink eingesetzt und unter Zusatz von etwas Aluminium als Reduktionsmittel legiert. Dazu werden Kupfer und Zink miteinander verschmolzen und die erzeugte Messingschmelze zu einem groben spratzigen Messinggrieß verdüst. Der erhaltene Messinggrieß wird anschließend zu Messingflakes vermahlen. Die Vermahlung von Messinggrieß erfolgt überwiegend nach dem Hametag'schen Trockenmahlverfahren. Hierbei wird der grobe Messinggrieß in Kugelmühlen in mehreren Mahlstufen bei unterschiedlichen Mahlbedingungen, wie beispielsweise Mühlengröße, - durchmesser, -drehgeschwindigkeit, Kugelgröße, Mahldauer unter Zugabe von Schmiermittel, wie beispielsweise Stearin- oder Ölsäure, zur Verhinderung einer Kaltverschweißung der Kupfer- bzw. Messingpartikel und mit Mahlhilfsmitteln, wie z. B. Stahlkugeln, vermahlen.

Zur Trockenvermahlung von Messinggrieß wird der als Mahlprodukt eingesetzte grobe spratzige Messinggrieß zu plättchenförmigen Messingpigmenten vermahlen. Die relativ schwer verformbaren Messingplättchen weisen eine etwa dreimal so hohe Dichte wie vergleichbare Aluminiumplättchen auf. Die Messingpigmente werden nach dem Vermahlen und Klassieren in verschiedenen Behältnissen gesammelt und anschließend homogenisiert. Um den späteren metallpigmentierten Beschichtungen den erforderlichen Metallglanz zu verleihen, können bei der anschließenden Nachbearbeitung zusätzliche Additive (wie z.B. Stearinsäure) auf die Oberfläche der Pigmentplättchen "aufpoliert" werden.

Bei Messingpigmenten (Goldbronzepigmenten) wird durch das Kupfer-Zink-Verhältnis der Farbton der Legierung bestimmt. In der Regel liegt der Kupfergehalt zwischen 70 und 100 Gew.-%. Goldbronzepigmente werden in charakteristischen Naturfarbtönen als "Bleichgold" mit einem Kupferanteil von ca. 90 Gew.-%, Rest Zink, als "Reichbleichgold" mit einem Kupferanteil von ca. 85 Gew.-%, Rest Zink und als "Reichgold" mit einem Kupferanteil von ca. 70 Gew.-%, Rest Zink, kommerziell gehandelt.

Die Herstellung von plättchenförmigen Messingpigmenten durch Vermahlung von Messinggrieß in Gegenwart von Mahlhilfsstoffen ist dem mit der Pigmentherstellung befassten Fachmann bekannt und wird beispielsweise in der DE 2007717 A beschrieben. Bei diesem Verfahren zur Herstellung von Messingpigmenten unter Einsatz einer inerten Flüssigkeit durchgeführten Nassvermahlung wird das Mahlgut in zumindest drei Kornfraktionen, d.h. in zwei Grobfraktionen und in eine Feinfraktion mit einer Korngröße kleiner 44 $\mu$m getrennt. Dabei wird die angefallene Feinfraktion gewonnen und die mittlere Grobfraktion aus dem Prozess entfernt sowie zumindest eine Grobfraktion mit dem Überkorn in die Mühle (Kugelmühle) zurückgeführt.

JP 63000406A zeigt ein Verfahren zur einfachen und kostengünstigen Herstellung von Metallpulver auf. Die Pulverteilchen bestehen aus durch mechanisches Vermahlen, z. B. in Kugelmühlen, unter Verwendung von Öl und Wasser erzeugten Metallflakes, z. B. auch aus Messing, mit einem hohen Formfaktor und einem normalen Pigmentdurchmesser sowie Dicke.

JP 2002327201A betrifft ein goldenes Tauchlackpulver aus Messingflakes mit einem durchschnittlichen Pigmentdurchmesser von 40-60 $\mu$m, einer Schüttdichte von 0,5-0,7 g/cm$^3$ und einem Spreitwert von mindestens 7000 cm$^2$/g.

US 2002891A betrifft die Herstellung eines Bronzepulvers aus Aluminium, Kupfer oder anderen Metallen und deren Legierungen. Das eingesetzte Metall wird unter definierten Mahlbedingungen zu flake-, plättchen- oder schuppenförmigen Pulverteilchen vermahlen.

Die US 3995815 A beschreibt ein Verfahren zur Herstellung von Flakes enthaltendem Metallpulver durch eine mittels Mischungsverhältnissen und Mahlzeiten definierte Nassvermahlung von Metallen in Kugelmühlen. Einzelheiten zu den Mahlprodukten sind dieser Druckschrift nicht zu entnehmen.

Der US 4172720 A ist ein flakeförmiges Metallpulver mit enger Dickenverteilung, reiner Farbe und mit einem sehr hohen Spiegeleffekt zu entnehmen. Dieses bekannte Metallpulver wird mittels einer durch bestimmte Gewichtsverhältnisse von Mahlhilfsmittel, Metall, Gleitmittel und Mahlflüssigkeit charakterisierten Nassvermahlung von Metall, beispielsweise auch von Messing, hergestellt.

Kupferflakes mit einer Dicke von unter 3 $\mu$m und einem Durchmesser unter 10 $\mu$m können nach dem in US 4,884,754 beschriebenen Verfahren hergestellt werden. Hierbei werden Kupferpartikel in Gegenwart von einem oder mehreren organischen Schmiermitteln in einem unpolaren organischen Lösungsmittel vermahlen. Anschließend wird der Hauptanteil an organischem Schmier- und Lösemittel entfernt und die erhaltenen Kupferflakes nochmals in einer Strahlmühle vermahlen.

Kupferflakes mit einem Durchmesser von 4 bis 10 $\mu$m und einem Formfaktor von 2 bis 20 für die Anwendung in beispielsweise elektrisch leitfähigen Pasten werden in US 2002/0050186 A1 beschrieben.

Dünne plättchenförmige Aluminiumpigmente mit enger Dickenverteilung sind aus WO 2008/077612 A2 bekannt. Diese Aluminiumpigmente weisen eine über Dickenauszählung mit Rasterelektronenmikroskopie ermittelte Dicke $h_{50}$ von 15 bis 75 nm auf.

Die WO 2004/026972 betrifft durch Vakuumabscheidung hergestellte kupferbasierende Metallflakes. Die US 6,398,861 B1 betrifft ein Verfahren zur Erstellung von Metalleffektpigmenten durch Vermahlung von Metallpulver. Die DE 103 15 775 A1 betrifft dünne deckende Aluminiumpigmente.

Der bestehende hohe Marktbedarf an Druckfarben, insbesondere für Tief- und Flexodruckfarben zur Herstellung von goldfarbenen spiegelartigen Effekten, kann derzeit nicht befriedigt werden.

Aufgabe der vorliegenden Erfindung ist es, plättchenförmige kupfer- bis goldfarbige Metalleffektpigmente mit verbesserten optischen Eigenschaften wie höherer Glanz und verbesserter Deckfähigkeit zur Verfügung zu stellen. Bei Druckfarben, insbesondere bei Tief- und Flexodruckfarben sollen die Metalleffektpigmente verbesserte optische Eigenschaften in Kombination mit einer verbesserten Spaltfestigkeit aufweisen.

Eine weitere Aufgabe bestand darin, plättchenförmige kupfer- bis goldfarbige Metalleffektpigmente, die auf einfache und kostengünstige Weise herzustellen sind, zur Verwendung von Folienkonterapplikationen mit spiegelartigem Effekt bereit zu stellen.

Die Aufgabe wird gelöst durch die Bereitstellung von plättchenförmigen kupferhaltigen Metalleffektpigmenten, die einen Kupfergehalt von 60 bis 100 Gew.-%, bezogen auf den gesamten Metallgehalt aufweisen, wobei sie eine über Dickenauszählung mit Rasterelektronenmikroskopie (REM) ermittelte Dickenverteilung in der Darstellung als Summendurchgangsverteilung,

    a) mit einem $h_{50}$-Wert von 10 bis 50 nm,

b) mit einem $h_{90}$-Wert von 20 bis 70 nm aufweisen,

wobei die plättchenförmigen kupferhaltigen Metalleffektpigmente durch Vermahlung eines kupferhaltigen Metallgrießes mit Schmiermittel hergestellt sind.

Bevorzugte Weiterbildungen der erfindungsgemäßen Metalteffektpigmente sind in den Unteransprüchen 2 bis 9 angegeben.

Die der Erfindung zugrunde liegende Aufgabe wird weiterhin durch ein Verfahren gemäß Anspruch 10 zur Herstellung von plättchenförmigen kupferhaltigen Metalleffektpigmenten nach einem der Ansprüche 1 bis 9 gelöst.

Bevorzugte Weiterbildungen des Verfahrens sind in den Unteransprüchen 11 bis 12 angegeben.

Die erfindungsgemäße Aufgabe wird ferner gelöst durch die Verwendung der erfindungsgemäßen Pigmente gemäß Anspruch 13 sowie durch eine die erfindungsgemäßen kupferhaltigen Metalleffektpigmente enthaltende Beschichtungszusammensetzung, vorzugsweise Druckfarbe, gemäß Anspruch 14.

Als erfindungsgemäße, plättchenförmige kupferhaltige Metalleffektpigmente sind im Rahmen der Erfindung Metalleffektpigmente, enthaltend oder bestehend aus Kupfer oder einer Legierung mit oder aus Zink und Kupfer (Messing), mit einem Kupfergehalt von wenigstens 60 Gew.-%, bezogen auf den gesamten Metallgehalt der Pigmente, zu verstehen.

Die erfindungsgemäßen kupferhaltigen Metalleffektpigmente umfassen Kupferpigmente sowie Messingpigmente (Goldbronzen).

Die Kupfereffektpigmente besitzen einen Kupfergehalt von 98 bis 100 Gew.-%, und bevorzugt von 99 bis 99,999 Gew.-%. Es versteht sich von selbst, dass der Fachmann bei der Angabe "100 Gew.-%" Kupfer auch einen sehr geringen Anteil gegebenenfalls vorhandener Fremdmetalle mitliest.

Die Messingpigmente, üblicherweise als "Goldbronzen" bezeichnet, weisen bevorzugt einen Kupfergehalt von 70 bis ca. 90 Gew.-% auf. Der Zinkgehalt liegt entsprechend zwischen 30 und 10 Gew.-%, wobei hier ebenfalls bis zu 2 Gew.-%, bevorzugt unter 1 Gew.-% Verunreinigungen anderer Metalle vorhanden sein können.

Eine weitere bevorzugte Ausführungsform nutzt sogar bewusst eine "Verunreinigung" mit beispielsweise 0,5 bis 2 Gew.-% Aluminiumgehalt, bezogen auf das gesamte Metalleffektpigment. Derartige Legierungen haben sich als korrosionsstabiler erwiesen.

Die erfindungsgemäßen, plättchenförmigen kupferhaltigen Metalleffektpigmente mit non-leafing Eigenschaften besitzen aufgrund ihrer sehr geringen mittleren Dicke eine sehr hohe Deckkraft. Als Deckkraft oder Deckvermögen eines Pigmentes wird üblicherweise die Abdeckung einer Fläche pro Gewichtseinheit an Pigmentmenge bezeichnet. Je dünner die mittlere Dicke der Pigmente ist, desto größer ist die durch das Pigment abgedeckte Fläche und mithin dessen Deckkraft.

Sehr dünne Metalleffektpigmente mit einer sehr engen Dickenverteilung stapeln sich gleichmäßiger im Anwendungsmedium als Metalleffektpigmente mit einer breiten Dickenverteilung. Bei den herkömmlichen Metalleffektpigmenten kann es leicht zu Ungleichmäßigkeiten in der Stapelung der Pigmente im Anwendungsmedium kommen. So können insbesondere sehr dicke Metalleffektpigmente als "Abstandshalter" wirken, welche die Orientierung der umgebenden bzw. benachbart liegenden Pigmente im Anwendungsmedium beeinträchtigt. Hiervon werden Glanz, Flop und unter Umständen das Deckvermögen der Metalleffektpigmente nachteilig beeinträchtigt. Dies wirkt sich insbesondere nachteilig in Druckanwendungen aus. Drucke haben im Vergleich zu Lackbeschichtungen (Coatings) eine wesentlich geringere Schichtdicke und einen geringeren Bindemittelanteil.

Die Bestimmung der exakten mittleren Dicke von plättchenförmigen Metallpigmenten ist schwierig. In der Praxis erfolgt die Pigmentdickenbestimmung über den Wasserbedeckungsgrad (Spreitung nach DIN 55923) oder durch Rasterelektronenmikroskopie (REM). Mit dem Wasserbedeckungsgrad lässt sich lediglich eine mittlere Dicke h der Pigmente berechnen, nicht aber die Dickenverteilung. Um auch die Dickenverteilung zu ermitteln, wurde im Rahmen dieser Erfindung die mittlere Dicke der erfindungsgemäßen Pigmente mittels Rasterelektronenmikroskopie (REM) bestimmt. Bei dieser Methode sind so viele Teilchen zu vermessen, dass eine repräsentative statistische Auswertung vorgenommen werden kann. Üblicherweise werden etwa 50 bis 100 Teilchen vermessen.

Die Dickenverteilung wird zweckmäßigerweise in Form einer Summendurchgangskurve bzw. Summenhäufigkeitsverteilung dargestellt. Als Mittelwert bietet sich der $h_{50}$-Wert der Dickensummendurchgangskurve an. Ein Maß für den Grobanteil ist der $h_{90}$-Wert. Er besagt, dass 90 % aller Pigmentteilchen eine Dicke gleich diesem Wert und/oder unterhalb dieses Wertes besitzen. Entsprechend besagt beispielsweise ein $h_{98}$-Wert, dass 98 % aller Pigmentteilchen eine Dicke gleich diesem Wert und/oder unterhalb dieses Wertes besitzen. In analoger Weise ist der $h_{10}$-Wert ein Maß für den Feinanteil der Dickenverteilung, der besagt, dass 10 % aller Pigmentteilchen eine Dicke gleich diesem Wert und/oder unterhalb dieses Wertes besitzen.

Der erfindungsgemäß verwendete Begriff "Summendurchgangsverteilung" wird auch als "Summenhäufigkeitsverteilung" bezeichnet. Diese beiden Begriffe sind mithin austauschbar verwendbar, weshalb in der vorliegenden Anmeldung anstelle des Begriffs "Summendurchgangsverteilung" auch der Begriff "Summenhäufigkeitsverteilung" verwendet werden kann.

Diese oben genannten Kennwerte können rechnerisch aus einer Liste der gemessenen Einzelwerte ermittelt werden, so z.B. mit Hilfe der "Quantil"-Funktion in einer Excel-Darstellung.
Die Ermittlung der Dicken der einzelnen Pigmente mittels REM erfolgt nach der in der DE 103 15 775 A1 beschriebenen Methode.

Im Ergebnis der Dickenauszählung mit Rasterelektronenmikroskopie ($h_{50}$-Wert der Summendurchgangs- bzw. Summenhäufigkeitsverteilung) wurde für die erfindungsgemäßen Goldbronzepigmente eine mittlere Dicke $h_{50}$ von 10 bis 50 nm, bevorzugt von 15 bis 45 nm, besonders bevorzugt von 15 bis 40 nm und ganz besonders bevorzugt von 20 bis 35 nm, ermittelt.

Unterhalb einer mittleren Dicke $h_{50}$ von 10 nm werden die resultierenden Farbtöne der Metalleffektpigmente zu dunkel, was auf eine Minderung des Reflexionsvermögens unter Beibehaltung der hohen Absorptionseigenschaften des Kupfers bzw. Messings zurückzuführen ist. Ebenso vermindert sich aufgrund der zunehmenden Transparenz der Metalleffektpigmente die Deckkraft und es können sich unerwünschte Farbtonverschiebungen einstellen.

Oberhalb einer mittleren Dicke $h_{50}$ von 50 nm waren bei den erfindungsgemäßen Metalleffektpigmenten vorteilhafte optische Eigenschaften nur noch in stark abgeschwächter Form vorhanden.

Weiterhin weisen die erfindungsgemäßen Metalleffektpigmente eine über Dickenauszählung mit Rasterelektronenmikroskopie (REM) ermittelte Dickenverteilung mit einem $h_{90}$-Wert von 20 bis 70 nm, bevorzugt von 20 bis 60 nm, weiter bevorzugt von 21 bis 50 nm und besonders bevorzugt von 22 bis 40 nm, auf.

Oberhalb eines $h_{90}$-Wertes von 70 nm waren die vorteilhaften Eigenschaften der erfindungsgemäßen Metalleffektpigmente nicht mehr zu beobachten. Insbesondere konnte ein klarer Spiegel in der Folienkonterapplikation (mit sehr guter Abbildschärfe) nicht mehr festgestellt werden.

Plättchenförmige kupferhaltige Metalleffektpigmente mit einem $h_{90}$-Wert von unter 20 nm konnten bislang nicht mittels Vermahlung hergestellt werden.

Es wird vermutet, dass die vorteilhaften optischen Eigenschaften der erfindungsgemäßen Metalleffektpigmente auf einer sehr geringen Dicke aller Pigmente in der Pigmentdickenverteilung beruhen. Deshalb sollte der $h_{98}$-Wert bevorzugt im Bereich von 21 bis unter 80 nm, besonders bevorzugt von 24 bis 70 nm und ganz besonders bevorzugt von 25 bis 60 nm liegen.

Vorteilhaft bewirken die geringen Dicken der erfindungsgemäßen kupferhaltigen Metalleffektpigmente eine sehr gute Orientierung der Pigmente im Anwendungsmedium, beispielsweise in einer Druckfarbe, insbesondere in einer Tief- und Flexodruckfarbe zur Herstellung von goldfarbigen Folienkonterapplikationen.
Es wird vermutet, dass ab einer bestimmten Plättchendicke diese derart flexibel werden, dass sie sich perfekt an den Untergrund anschmiegen. Dieser Effekt ist von PVD-Aluminiumpigmenten seit langem bekannt und wird insbesondere in den Folienkonterapplikationen ausgenutzt.

Bei einer weiterhin bevorzugten Ausführungsform der Erfindung weisen die erfindungsgemäßen Metalleffektpigmente einen $h_{10}$-Wert der Dickenverteilung im Bereich von 8 bis 25 nm und besonders bevorzugt von 10 bis 20 nm auf. Unterhalb eines $h_{10}$-Wertes von 8 nm sind die Pigmente zu dünn, was zu verschlechterten optischen Eigenschaften führt. Oberhalb eines $h_{10}$-Wertes von 25 nm sind die Pigmente wiederum zu dick, da bei hohen $h_{10}$-Werten auch entsprechend die $h_{50}$-und $h_{90}$-Werte größer sind.

Weiterhin weisen die erfindungsgemäßen Metalleffektpigmente eine über Dickenauszählung mit Rasterelektronenmikroskopie (REM) ermittelte relative Breite der Dickenverteilung Δh, welche anhand der entsprechenden Summendurchgangskurve der relativen Häufigkeit nach der Formel

$$\Delta h = 100 \times (h_{90}-h_{10}) / h_{50}$$

berechnet wird, von 30% bis 90%, bevorzugt von 35% bis 85% und besonders bevorzugt von 40% bis 80%, auf.

Aufgrund der in überraschender Weise PVD-Metalleffektpigmenten ähnlichen, engen Dickenverteilung der durch Nassvermahlung hergestellten, erfindungsgemäßen Metalleffektpigmente ähneln diese in ihren optischen Eigenschaften PVD-Pigmenten, sind jedoch wesentlich kostengünstiger herzustellen.

In der Längsausdehnung unterscheiden sich die erfindungsgemäßen, insbesondere durch Nassvermahlung von Kupfer- oder Messinggrieß hergestellten Metalleffektpigmente nicht grundsätzlich von kommerziell gehandelten, durch Trockenvermahlung von Kupfer- oder Messinggrieß hergestellten Goldbronzepigmenten. Im Einzelnen hängen die Pigmentgrößen vom Einsatzzweck ab.

Die erfindungsgemäßen kupferhaltigen Metalleffektpigmente weisen bevorzugt eine mittlere Größe $d_{50}$ von 3 bis 50 μm, weiter bevorzugt von 4 bis 30 μm, besonders bevorzugt von 5 bis 20 μm und ganz besonders bevorzugt von 6 bis 15 μm, auf. Die Längsausdehnung d (Durchmesser) wird in Laserbeugungsexperimenten auf Grundlage der Fraunhofer- und/oder der Miebeugungstheorie bestimmt. Der Auswertung der Beugungsdaten liegt ein Modell zugrunde, welches auf den Durchmesser einer Äquivalentkugel abzielt. Daher werden keine Absolutwerte erhalten, jedoch haben sich die gemessenen Durchmesser als verlässliche Relativwerte in der Beschreibung der Größencharakteristik von plättchenförmigen Metallpigmenten durchgesetzt.

Der $d_{50}$-Wert der Pigmentlänge entspricht dabei 50% der Summendurchgangsverteilungskurve, gemessen und ausgewertet in Form einer Volumenverteilung von Äquivalentkugeln.

Völlig überraschend wurde festgestellt, dass die mit erfindungsgemäßen kupferhaltigen Metalleffektpigmenten pigmentierten Beschichtungszusammensetzungen bei "Folienkonterapplikationen" einen goldfarbenen spiegelartigen Effekt aufweisen, der mit herkömmlichen, durch Trockenvermahlung hergestellten kupferhaltigen Metalleffektpigmenten bisher nicht realisiert werden konnte.

Unter einer "Folienkonterapplikation" wird verstanden, dass eine mit Metalleffektpigmenten pigmentierte Druckfarbe auf eine transparente Folie aufgedruckt wird. Der auf der Folie ausgehärtete Druck ergibt bei Verwendung von erfindungsgemäßen kupferhaltigen Metalleffektpigmenten einen goldfarbenen (goldfarbigen) spiegelartigen Effekt in der Konteransicht. Dieser Spiegeleffekt entsteht dadurch, dass sich die erfindungsgemäßen kupferhaltigen Metalleffektpigmente aufgrund ihrer geringen Dicke und ihrer engen Dickenverteilung, sowie aufgrund ihrer non-leafing Eigenschaft direkt an der Folienoberfläche ausrichten.

Als treibende Kraft für eine planparallele Orientierung von Metalleffektpigmenten ist, neben der grenzflächenchemischen Unverträglichkeit der Pigmente zum Bindemittelsystem, der Formfaktor ein weiteres wichtiges Charakteristikum für die Eigenschaften der erfindungsgemäßen Metalleffektpigmente.

Unter dem Formfaktor f versteht man das Verhältnis des Mittelwertes der Längsausdehnung zur mittleren Dicke der Pigmentplättchen.

Der dimensionslose Formfaktor f in dieser Erfindung ist definiert als:

$$f = 1000 * \frac{d_{50}(\mu m)}{h_{50(nm)}}$$

Die erfindungsgemäßen kupferhaltigen Metalleffektpigmente (Goldbronzepigmente) weisen vorzugsweise einen Formfaktor f von 150 bis 3.000 auf. Bevorzugt sind die erfindungsgemäßen Pigmente durch einen Formfaktor f von 250 bis 2.500, weiter bevorzugt von 300 bis 1.000 und besonders bevorzugt von 325 bis 600, gekennzeichnet.

Bei Drucken sind die Bindemittelanteile und die Schichtdicken generell sehr viel niedriger als in Lacken. Dies trifft vor allem für Tiefdruckfarben zu. Mit kommerziell gehandelten Goldbronzepigmenten pigmentierte Tiefdruckfarben weisen einen Festkörpergehalt von ca. 40 Gew.-% auf. Druckfilme hieraus weisen eine Nassfilmschichtdicke von ca. 3 bis 6 $\mu$m und eine Trockenfilmschichtdicke von ca. 1,5 bis 3 $\mu$m auf. Im Fall von mit PVD-Pigmenten pigmentierten Tiefdruckfarben liegen die Festkörperanteile bei ca. 5 bis 20 Gew.-% der gesamten Tiefdruckfarbe. Damit gehen Trockenfilmschichtdicken von nur 0,5 bis 1,5 $\mu$m einher. Bei diesen äußerst geringen Schichtdicken ist eine weitgehend gleichförmige planparallele Orientierung der Metallpigmente notwendig. Diese konnte bisher nur mit PVD-Metalleffektpigmenten erreicht werden.

Mit den erfindungsgemäßen kupferhaltigen Metalleffektpigmenten pigmentierte Druckanwendungen, insbesondere Folienkonterapplikationen, weisen aufgrund der geringen mittleren Teilchendicke und der engen Teilchendickenverteilung der erfindungsgemäßen Pigmente vergleichbare optische Effekte (bzgl. Glanz / Spiegel) wie bei mit herkömmlichen PVD-Metalleffektpigmenten pigmentierten Druckanwendungen auf.

Bei einer weiteren bevorzugten Ausführungsform der Erfindung ist die Oberfläche der erfindungsgemäßen Metalleffektpigmente wenigstens teilweise mit einem Additiv bedeckt, wobei das Additiv als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind.

Unter "Struktureinheiten" wird erfindungsgemäß verstanden, dass das Additiv eine Carbonsäure mit wenigstens 4 Kohlenstoffatomen umfaßt. Die Carbonsäure kann dabei als solche oder als Substituent, beispielsweise in Form einer Seitenkette, vorliegen. Wesentlich ist, dass das verwendete Additiv jedenfalls wenigstens eine Struktureinheit in Form einer Carbonsäure mit wenigstens 4 Kohlenstoffatomen aufweist.

Additive dieses Typs haben sich überraschenderweise als hervorragende Schmiermittel zur Vermahlung von kupferhaltigem Metallgrieß zu Metalleffektpigmenten erwiesen.

Dabei ist bei dem Additiv bevorzugt der Polyglykolether mit der wenigstens einen Carbonsäure miteinander verestert.

Gemäß einer weiteren bevorzugten Ausführungsform werden als Carbonsäuren Dicarbonsäuren, Tricarbonsäuren, Tetracarbonsäuen oder deren Mischungen verwendet. Als Di- und/oder Tricarbonsäuren, die mit Polyglykolether kovalent verbunden werden können, können ebenfalls gesättigte und/oder ungesättigte Carbonsäuren verwendet werden.

Beispielsweise können als Dicarbonsäuren Bernsteinsäure, Glutarsäure, Adipinsäure, Pimelinsäure, Korksäure, Azelainsäure und/oder Sebacinsäure verwenden werden.

Gemäß einer bevorzugten Weiterbildung der Erfindung werden Di-, Tri- oder Tetracarbonsäuren mit längeren Kohlenstoffgerüsten verwendet, beispielsweise mit 11 bis 30 Kohlenstoffatomen, vorzugsweise mit 12 bis 24 Kohlenstoffatomen, noch weiter bevorzugt mit 14 bis 22 Kohlenstoffatomen. Diese Di-, Tri- oder Tetracarbonsäuren entstehen bevorzugt durch Di-, Tri- oder Tetramerisierung ungesättigter Fettsäuren wie beispielsweise Ölsäure, Linolsäure, Linolensäure, Eleostearinsäure oder ähnlichen Säuren.

Als sehr geeignet haben sich Dicarbonsäuren mit einem Kohlenstoffgrundgerüst von 18 Kohlenstoffatomen erwiesen. Diese Dicarbonsäure weist demnach 36 C-Atome und die entsprechende Tricarbonsäure 54 C-Atome auf. Vorzugsweise werden Mischungen dieser verschiedenen Fettsäuren verwendet.

Hinsichtlich der Polyetherstruktureinheit des Additivs umfasst gemäß einer bevorzugten Weiterbildung der Erfindung der Polyglykolether die Gruppe $R^1$-O-$(R^2$-O$)_y$-$(R^3$-O$)_z$-$(R^4$-O$)_k$-, wobei die $R^2$-O-, $R^3$-O- und die $R^4$-O-Polyethereinheiten statistisch, alternierend oder als Blockcopolymere angeordnet sein können. Hierbei ist $R^1$ ein linearer oder verzweigter aliphatischer Rest oder araliphatischer oder aromatischer organischer Rest mit 1 bis 30 Kohlenstoffatomen. Die Reste $R^2$, $R^3$ und $R^4$ können gleich oder unabhängig voneinander verschieden sein und stehen jeweils für einen linearen oder verzweigten aliphatischen organischen Rest oder araliphatischen oder aromatischen organischen Rest mit 1 bis 12 Kohlenstoffatomen. Die Einzelpolymerisationsgrade y, z und k stehen unabhängig voneinander für 0 bis 200, mit der Maßgabe, daß der Gesamtpolymerisationsgrad y+z+k = 2 bis 600 ist.

Der Rest $R^1$ ist bevorzugt ein linearer oder verzweigter aliphatischer Rest oder araliphatischer oder aromatischer organischer Rest mit 2 bis 16 Kohlenstoffatomen und besonders bevorzugt ein aliphatischer Rest mit 1 bis 12 C-Atomen.

Die Reste $R^2$, $R^3$ und $R^4$ weisen bevorzugt unabhängig voneinander 2 bis 8 C-Atome und besonders bevorzugt 2 bis 4 C-Atome auf.

Besonders bevorzugt handelt es sich bei den Resten $R^2$, $R^3$ und $R^4$ unabhängig voneinander um Ethyl, iso-Propyl, -Propyl oder Butyl. Weiter besonders bevorzugt sind alternierende Ethyl, iso-Propyl-Einheiten, sogenannte EO/PO-Polyether.

Die Länge der Ethereinheiten (Gesamtpolymerisationsgrad) y+z+k ist bevorzugt 5 bis 300, weiter bevorzugt 7 bis 100 und besonders bevorzugt 10 bis 50.

Entsprechende Verbindungen finden sich beispielsweise in der EP 1 304 210 A1. Hier sind jedoch die Verbindungen nur als Prozessmittel für Kunststoffe und nicht als Schmiermittel für die Herstellung von Metalleffektpigmenten beschrieben.

Weiterhin können die erfindungsgemäßen Metalleffektpigmente eine Metalloxidschicht aufweisen, wobei das Metall der Metalloxidschicht von gleicher Art wie das Metall des Metalleffektpigmentes ist.

Derartige Oxidschichten kennt man auch von den üblichen Kupfer- bzw.

Goldbronzepigmenten. Diese Oxidschichten werden durch sogenannte Feuerfärbungen erhalten. Aufgrund ihrer Eigenfarben und von Interferenzeffekten ergeben diese Metalloxidschichten in Abhängigkeit von ihrer Schichtdicke Effektpigmente unterschiedlichster Farbtöne im gelb-roten Farbbereich. Selbstverständlich spielt hierbei auch die Grundfarbe des Metallpigmentes eine große Rolle.

Da sich bei der Oxidation naturgemäß stets ein Teil des Metalls in das entsprechende Oxid umwandelt, ist es bevorzugt, nicht die dünnsten erfindungsgemäßen Metallpigmente zur Oxidation heran zu ziehen. Diese hätten nur noch einen sehr geringen bis gar keinen Metallgehalt mehr, was große Nachteile in Bezug auf ihre Deckkraft hätte. Daher werden zur Oxidation ("Feuerfärbung") nur erfindungsgemäße Metallpigmente mit mittleren Dicken $h_{50}$ im Bereich von 25 bis 50 nm verwendet.

Die Erfindung betrifft mithin ferner feuergefärbte kupferhaltige Metalleffektpigmente.

Zur Verwendung in Wasser enthaltenden Medien, beispielsweise in wässrigen Druckfarben oder witterungsbeständigen Außenanwendungen, können die erfindungsgemäßen Metalleffektpigmente mit einer passivierenden Inhibitor- und/oder passivierenden Korrosionsschutzschicht belegt sein.

Der Wirkungsmechanismus der Inhibitoren- und/oder Passivierungsschichten ist komplex.

Bei Inhibitoren beruht die inhibierende oder passivierende Wirkung zumeist auf sterischen Effekten. Der größte Teil der Inhibitoren hat daher auch eine orientierende Wirkung im Sinne von "leafing" und "non-leafing", d.h. im Anwendungsmedium aufschwimmend bzw. nicht aufschwimmend.

Die Inhibitoren werden üblicherweise in niedrigen Konzentrationen in der Größenordnung von 1 Gew.-% bis 15 Gew.-%, bezogen auf das Gewicht des eingesetzten Metalleffektpigmentes, zugegeben.

Als passivierende Inhibitoren für die erfindungsgemäßen Metalleffektpigmente können Fettsäuren, Carbonsäurederivate, organische Phosphate und Phosphonate und deren Ester, organisch funktionalisierte Silane, aliphatische oder cyclische Amine, aliphatische und aromatische Nitroverbindungen, Sauerstoff-, Schwefel- oder Stickstoff-enthaltende Heterocyclen, Schwefel-/Stickstoffverbindungen höherer Ketone, Aldehyde und Alkohole, Thiole, β-Diketone, β-Ketoester oder deren Gemische, Verwendung finden.

Vorzugsweise kommen für die Inhibierung der erfindungsgemäßen Metalleffektpigmente in Frage:

- Organisch modifizierte Phosphonsäuren bzw. deren Ester der allgemeinen Formel R-P(O)(OR$_1$)(OR$_2$), wobei: R = Alkyl, Aryl, Alkyl-aryl, Aryl-alkyl sowie Alkylether, insbesondere ethoxylierte Alkylether und R$_1$, R$_2$ = H, C$_n$H$_{2n+1}$, mit n = 1-6 ist, wobei Alkyl jeweils verzweigt oder unverzweigt sein kann. R$_1$ kann gleich oder unterschiedlich zu R$_2$ sein.

- Organisch modifizierte Phosphorsäuren und -ester der allgemeinen Formel. R-O-P(OR$_1$)(OR$_2$) mit R = Alkyl, Aryl, Alkyl-aryl, Aryl-alkyl sowie Alkylether, insbesondere ethoxylierte Alkylether und R$_1$, R$_2$= H, C$_n$H$_{2n+1}$, mit n = 1-6 ist, wobei Alkyl jeweils verzweigt oder unverzweigt sein kann. R$_1$ kann gleich oder unterschiedlich zu R$_2$ sein.

Verwendet werden können reine Phosphonsäuren oder -ester oder Phosphorsäuren oder -ester oder beliebige Mischungen derselben.

Im Falle einer Vermahlung der erfindungsgemäßen Metalleffektpigmente in einem überwiegend wässrigen Lösemittel werden derartige Inhibitoren als Mahlhilfsmittel verwendet, um einer sicherheitstechnisch gefährlichen Wasserstoffentstehung während des Mahlvorganges vorzubeugen.

Weiterhin kann die passivierende Inhibitorschicht korrosionsinhibierende organisch funktionalisierte Silane, aliphatische oder cyclische Amine, aliphatische oder aromatische Nitroverbindungen oder Sauerstoff-, Schwefel- und/oder Stickstoff enthaltende Heterocyclen umfassen oder daraus bestehen. Hierbei sind stickstoffhaltige heterocyclische Verbindungen bevorzugt, besonders bevorzugt sind Triazole und ganz besonders bevorzugt sind Benzotriazole. Weiterhin können beispielsweise Thioharnstoffderivate, Schwefel- und/oder Stickstoffverbindungen höherer Ketone, Aldehyde und Alkohole (Fettalkohole) oder Thiole, oder Gemische derselben verwendet werden. Die passivierende Inhibitorschicht kann aber auch aus den vorgenannten Substanzen bestehen. Bevorzugt sind organische Phosphonsäuren und/oder Phosphorsäureester oder deren Gemische. Bei Verwendung von Aminverbindungen weisen diese bevorzugt organische Reste mit mehr als 6 C-Atomen auf. Bevorzugt werden derartige Amine zusammen mit organischen Phosphonsäuren und/oder Phosphorsäureester oder deren Gemische eingesetzt.

Die Passivierung von Metalleffektpigmenten durch Korrosionsschutzbarrieren mit chemischer und physikalischer Schutzwirkung ist auf vielfältige Weise möglich.

Passivierende Korrosionsschutzschichten, die den erfindungsgemäßen Metalleffektpigmenten einen besonders guten Korrosionsschutz gewährleisten, umfassen oder bestehen aus Siliziumoxid, bevorzugt Siliziumdioxid, Zirkoniumoxid, Ceroxid, Aluminiumoxid, polymerisierte Kunststoffharze, Phosphate, Phosphite, Borate oder Mischungen derselben.

Bevorzugt sind Siliciumdioxid-Schichten, wobei die Siliziumdioxid-Oberfläche vorzugsweise mit Silanen belegt ist

Die SiO$_2$-Schichten werden bevorzugt durch Sol-Gel-Verfahren mit durchschnittlichen Schichtdicken von 2 bis 150 nm und bevorzugt von 5 bis 40 nm in organischen Lösemitteln hergestellt.

Die erfindungsgemäßen kupferhaltigen Metalleffektpigmente werden bevorzugt in Form von Pigmentpaste, besonders bevorzugt in Form eines pastenförmigen Produkts für Tief- und Flexodruckfarben, eingesetzt.

Im Folgenden wird das Verfahren zur Herstellung der erfindungsgemäßen plättchenförmigen kupferhaltigen Metalleffektpigmente dargestellt.

Die erfindungsgemäßen kupferhaltigen Metalleffektpigmente werden durch Vermahlung eines kupferhaltigen Metallgrießes mit Schmiermittel hergestellt. Bevorzugt handelt es sich um eine Naßvermahlung. Der kupferhaltige Metallgrieß ist bevorzugt ein Kupfer- oder Messinggrieß.

Es handelt sich weiter bevorzugt um eine Vermahlung, die einen schonenden Verformungsmahlschritt von Kupfer- oder Messinggrieß umfasst. Der Kupfer- oder Messinggrieß weist weiter bevorzugt eine sehr enge Korngrößenverteilung auf.

Ein Verfahren zur Herstellung von plättchenförmigen kupferhaltigen Metalleffektpigmenten umfasst folgenden Schritt:

Vermahlen eines kupferhaltigen Metallgrießes mit einer Korngrößenverteilung mit einem d$_{Grieß,50}$ von 1 bis 15 $\mu$m und einem d$_{Grieß,90}$ von 2 bis 27 $\mu$m und einem Kupfergehalt von 60 bis 100 Gew.-%, bezogen auf den gesamten Metallgrieß zu plättchenförmigen Metalleffektpigmenten unter Verwendung eines Mahlwerks in Gegenwart von Schmiermitteln und Mahlkörpern und optional Lösemittel, wobei die plättchenfömigen Metalleffektpigmente eine über Dickenauszählung mit Rasterelektronenmikroskopie (REM) ermittelte mittlere Dicke mit einem h$_{50}$-Wert von 10 bis 50 nm und einem h$_{90}$-Wert von 20 bis 70 nm aufweisen, wobei der kupferhaltige Metallgrieß ein Kupfer- oder Messinggrieß ist und einen Span $\Delta d_{Grieß} = (d_{Grieß,90} - d_{Grieß,10})/d_{Grieß,50}$ von 0,8 bis 1,7 aufweist.

Bei der Vermahlung handelt es sich besonders bevorzugt um eine Naßvermahlung in Gegenwart von Lösemittel.

Bei einer weiteren bevorzugten Ausführungsform der Erfindung werden die Grießpartikel in zwei Stufen vermahlen. Dabei werden in der ersten Stufe die Grießpartikel vorverformt und in der zweiten Stufe ausgemahlen.

Der Vorverformungsschritt wird dabei unter Bedingungen, die einen höheren Energieeintrag auf die Metallpartikel ermöglichen, durchgeführt.

Die beiden Stufen können beispielsweise mit unterschiedlichen Kugelgrößen durchgeführt werden. Zweckmäßigerweise wird man hierbei in dem Vorverformungsschritt größere Kugeln, mit denen ein höherer Energieeintrag möglich ist, auswählen.

Bei diesem Verfahren muß man jedoch in zwei Stufen arbeiten, was relativ aufwändig ist.

Weiter bevorzugt werden daher die beiden Stufen in einer Mühle mit derselben Mahlkörperpackung durchgeführt. Dabei kann der unterschiedliche Energieeintrag beispielsweise durch verschiedene Umdrehungsgeschwindigkeiten der Mühle und/oder durch verschiedene Mahldauern eingestellt werden.

Bei weiter bevorzugten Ausführungsformen weist der kupferhaltige Metallgrieß einen $d_{\text{Grieß},50}$ von 1,5 bis 10 $\mu$m und besonders bevorzugt von 2 bis 5 $\mu$m auf.

Weiterhin ist bevorzugt, dass der kupferhaltige Metallgrieß einen $d_{\text{Grieß},90}$ von 2,5 bis 20 $\mu$m und besonders bevorzugt von 3 bis 7 $\mu$m aufweist.

Weiterhin weist der erfindungsgemäß eingesetzte Kupfer- oder Messinggrieß bevorzugt einen Span $\Delta d_{\text{Grieß}} = (d_{\text{Grieß},90}-d_{\text{Grieß},10})/d_{\text{Grieß},50}$ von 0,8 bis 1,7 und besonders bevorzugt von 0,9 bis 1,3 auf.

Dieser Kupfer- oder Messinggrieß ist ein sehr feiner Metallgrieß mit einer sehr engen Größenverteilung. Das Kornband der Größenverteilung wird in üblicher Weise durch Laserbeugungsspektrometrie ermittelt, wobei aus der Laserlichtbeugung die Partikelgröße ermittelt werden kann. Die Laserbeugungsspektrometrie wird dabei bevorzugt mit dem Gerät Helos der Firma Sympatec GmbH, Clausthal-Zellerfeld, Deutschland, gemäß Herstellerangaben durchgeführt.

Der zur Herstellung der erfindungsgemäßen kupferhaltigen Metalleffektpigmente eingesetzte Metallgrieß wird bevorzugt in "Atomizern" durch Verdüsung von flüssigem Kupfer oder einer Kupfer-Zink-Legierung, also Messing, vorzugsweise aus einer Kupfer- oder Messingschmelze, hergestellt. Der nach Verdüsung einer Kupfer- oder Messingschmelze erhaltene Grieß wird gemäß einer bevorzugten Variante klassiert, um die gewünschte Korngrößenverteilung, die auch als Kornband bezeichnet werden kann, zu erhalten.

Der Kupfer- oder Messinggrieß kann nach dem Verdüsungsschritt durch entsprechende Klassierungsschritte auf die gewünschte enge Größenverteilung gebracht werden. Die Klassierung kann mit Windsichtern, Zyklonen und anderen bekannten Einrichtungen durchgeführt werden.

Die Verwendung eines derartig feinen Kupfer- oder Messinggrießes mit enger Größenverteilung ist von essentieller Bedeutung für die Herstellung der erfindungsgemäßen, plättchenförmigen kupferhaltigen Metalleffektpigmente.

Während der Verformungsmahlung werden die Kupfer- oder Messinggrießpartikel nicht völlig gleichmäßig verformt: Dies bedeutet, dass einige Metallpartikel stärker verformt werden, während ein Teil der Grießpartikel erst sehr spät während der Vermahlung verformt wird. Ein Grund hierfür ist auch die Tatsache, dass die Verformungswahrscheinlichkeit eines Metallpartikels von seiner Größe abhängt. So besitzen bereits zu Plättchen vorverformte Metallpartikel eine höhere spezifische Fläche als noch unverformter Metallgrieß und demgemäß eine höhere Wahrscheinlichkeit, weiter verformt zu werden. Die Breite der Größenverteilung des Metallgrießes geht somit nicht nur in die Größenverteilung der daraus geformten Kupfer- oder Messingplättchen ein, sondern auch in die Verteilung der Dickenverteilung. Für enge Dickenverteilungen muss daher ein Kupfer- oder Messinggrieß mit entsprechend geringer Größenvarianz verwendet werden.

Der Verdüsungsschritt kann in Luftatmosphäre oder unter Inertgasatmosphäre durchgeführt werden. Als Inertgase werden vorzugsweise Stickstoff und/oder Helium eingesetzt.

Die Reinheit des bei der Verdüsung verwendeten Kupfers oder der Kupfer-Zink-Legierung (Messing) beträgt vorzugsweise 99,0 bis über 99,9 Gew.-%. Der Grieß kann in entsprechend geringen Mengen die üblichen Legierungsbestandteile (z.B. Al, Si, Fe, Sn, Pb) enthalten. Bevorzugt werden 0,1 - 2 Gew.-% Aluminium zulegiert.

Die Nassvermahlung des erfindungsgemäßen Kupfer- oder Messinggrießes erfolgt in herkömmlichen Mühlen, vorzugsweise in einer Kugelmühle, Rührwerkskugelmühle, Kollermühle, Trommelkugelmühle oder Drehrohrkugelmühle, in Gegenwart von Lösemittel und Schmiermitteln als Mahlhilfsmittel sowie unter Verwendung von Mahlkörpern.

Bei der in wenigstens zwei Schritten erfolgenden Nassvermahlung des erfindungsgemäßen Kupfer- oder Messinggrießes werden Mahlkörper, vorzugsweise kugelförmige Mahlkörper mit einem mittleren Durchmesser von 0,3 bis zu 4,7 mm und bevorzugt von 0,6 bis 2 mm eingesetzt.

Die in vielfältigen Ausführungsformen, wie beispielsweise Kugeln, Ellipsoide, Zylinder, Quader etc., eingesetzten Mahlkörper bestehen bevorzugt aus Chromstahl, Stahl, Glas oder Keramik. Besonders bevorzugt bestehen die Mahlkörper aus Chromstahl. Weiterhin werden besonders bevorzugt als Mahlkörper vorzugsweise sphärische Körper, weiter bevorzugt Kugeln, verwendet.

Bevorzugt sind kugelförmige Mahlkörper mit sehr glatter Oberfläche, möglichst runder Form und einheitlicher Größe.

Die zur Nassvermahlung des Kupfer- oder Messinggrießes eingesetzten Mahlkörper weisen vorzugsweise ein Einzelgewicht von 85 $\mu$g bis 425 mg auf.

Gemäß einer bevorzugten Weiterbildung der Erfindung weisen die Mahlkörper ein Einzelgewicht von 0,8 bis 180 mg auf.

Im Falle von Stahlkugeln liegt das mittlere Einzelgewicht vorzugsweise in einem Bereich von 1 bis 180 mg, vorzugsweise von 1,2 bis 150 mg, weiter bevorzugt von 2,0 bis 120 mg. Im Falle von Glaskugeln liegt das mittlere Einzelgewicht in einem Bereich von 1,0 bis 12,5 mg.

Aufgrund der äußerst schonenden Mahlweise dauert diese Vermahlung vergleichsweise lang.

Die Mahldauer beträgt vorzugsweise 10 bis 100 Stunden, bevorzugt 20 bis 60 Stunden und besonders bevorzugt 30 bis 50 Stunden.

Diese Zeiten verstehen sich als Gesamtmahldauerzeiten.

Wenn die Mahlung in zwei oder mehr verschiedenen Schritten durchgeführt wird, so sind entsprechend die Mahldauern der einzelnen Schritte zu addieren.

Diese langen Mahldauern führen zu einer Vielzahl von Pigment-Mahlkörper-Stößen. Dadurch wird das Pigment sehr gleichmäßig ausgeformt, wodurch eine sehr glatte Oberfläche und eine sehr enge Dickenverteilung ausgebildet werden.

Dies ist bei einer Mahldauer von weniger als 10 Stunden in der Regel nicht zu erreichen. Mahldauern oberhalb von 100 Stunden hingegen sind mehr und mehr unwirtschaftlich.

Die Temperaturen während des Mahlvorganges liegen im Bereich von 15°C bis 55°C. Bevorzugt sind Temperaturen in einem Bereich von 20°C bis 35°C.

Bei der Vermahlung wird Kupfer- oder Messingpulver mit bestimmter Teilchengröße zusammen mit Lösemittel, z. B. Testbenzin, in eine Kugelmühle eingetragen.

Als Lösemittel können handelsübliche, organische Lösemittel, vorzugsweise Testbenzin, Solventnaphtha, Alkohole, Glykole, Ester, Ether, Ketone oder Mischungen davon verwendet werden.

Bevorzugt sollte die Vermahlung in Lösemitteln durchgeführt werden, die kompatibel zu der später geplanten Anwendung sind.

So sind beispielsweise für eine Anwendung in einer Tiefdruckfarbe Lösemittel wie Ethylacetat, n-Propylacetat oder iso-Propylacetat bevorzugt.

Der üblicherweise bei Aluminiumpigmenten praktizierte Umnetzungsschritt ist hier nicht zu empfehlen. Beim Umnetzen werden, wenn sich dies als erforderlich erweist, die Metalleffektpigmente nach der Vermahlung unter vermindertem Druck und erhöhten Temperaturen von ihrem Lösemittel weitgehend befreit und anschließend mit dem für die jeweilige Endanwendung kompatiblen (und vom Kunden gewünschten) Lösemittel wieder angepastet.

Aufgrund der sehr hohen spezifischen Oberflächen der erfindungsgemäßen Metalleffektpigmente kann es bei dem Umnetzungsschritt zu unerwünschten Agglomerationen der Metallpigmente kommen. Daher sollte bevorzugt in Lösemitteln vermahlen werden, die kompatibel zu der später geplanten Anwendung sind.

Ebenfalls kann Wasser (im zumindest überwiegenden Teil) als Lösemittel verwendet werden. In diesem Fall sollten die eingesetzten Schmiermittel allerdings deutlich korrosionsinhibierende Wirkung haben. Bevorzugt sind hier Phosphonsäuren und/oder Phosphorsäureester, die auch ethoxylierte Seitenketten tragen können. Auch ist die Zugabe von weiteren Korrosionsinhibitoren während der Mahlung hier zu empfehlen.

Die Mahlung wird vorzugsweise in einem Lösemittel bei einem Gewichtsverhältnis von Lösemittel zu Metallpartikel von vorzugsweise 1,5:1 bis 5:1 und besonders bevorzugt von 2:1 bis 4:1 durchgeführt.

Zur Vermeidung des Kaltverschweißens der Pulverpartikel wird Schmiermittel, beispielsweise Ölsäure, Stearinsäure oder auch Inhibitoren, in einer Menge zugesetzt, welche von der jeweiligen freien spezifischen Oberfläche (BET) der ausgewalzten Kupfer- oder Messingpigmente abhängig ist. In der Regel werden 1 bis 30 Gew.-% und bevorzugt 1,5 - 10 Gew.-% Schmiermittel, bezogen auf das Gewicht des Kupfer- oder Messinggrießes, eingesetzt.

Als Schmiermittel können während der Vermahlung eine Vielzahl von Verbindungen verwendet werden.

Hierbei sind vor allem die seit langer Zeit verwendeten Fettsäuren mit Alkylresten von 10 bis 24 C-Atomen zu nennen. Vorzugsweise wird Ölsäure oder Mischungen verschiedener ungesättigter Fettsäuren oder Mischungen von ungesättigten und gesättigten Fettsäuren verwendet, die zu non-leafing-Pigmenten führen. Non-leafing-Pigmente ordnen sich in einem Anwendungsmedium, beispielsweise einem Lack oder einer Druckfarbe - im Gegensatz zu im Anwendungsmedium an die Oberfläche aufschwimmenden leafing-Pigmenten - an. Den Fettsäuren können zusätzlich beispielsweise langkettige Aminoverbindungen zugesetzt werden. Die Fettsäuren können tierischen oder auch pflanzlichen Ursprungs sein.

Das Schmiermittel sollte in nicht zu geringer Menge eingesetzt werden, da anderenfalls infolge der starken Ausformung des Kupfer- oder Messinggrießes die sehr großen Oberflächen der hergestellten plättchenartigen Kupfer- oder Messingpigmente nur ungenügend durch adsorbiertes Schmiermittel abgesättigt werden. In diesem Fall kommt es zu Kaltverschweißungen. Typische Mengen sind daher 1 bis 30 Gew.-%, bevorzugt 2 bis 15 Gew.-% Schmiermittel bezogen auf das Gewicht des eingesetzten Kupfer- oder Messinggrießes.

Als besonders bevorzugtes Schmiermittel wird ein Additiv verwendet, wobei das Additiv als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind.

Hierbei ist besonders bevorzugt, dass die Carbonsäuren oder Fettsäuren mit einem Polyglykolether zumindest teilweise verestert sind.

So kann beispielsweise das als Prozeßhilfsmittel für Kunststoffe im Handel erhältliche Fettsäurepolyglykolester "P4100" der Fa. BYK-Chemie, Wesel, Deutschland, eingesetzt werden.

Das Gewichtsverhältnis Mahlkugeln zu Metallpartikeln beträgt vorzugsweise 10 zu 1 bis 60 zu 1, besonders bevorzugt von 25 zu 1 bis 50 zu 1.

Bezogen auf die Vermahlung in einer Kugelmühle ist die kritische Drehzahl $n_{krit}$ ein wichtiger Parameter, der angibt, ab wann die Kugeln durch die Zentrifugalkräfte an die Mühlenwand gepresst werden und praktisch keine Mahlung mehr stattfindet:

$$n_{krit} = \sqrt{\frac{g}{2\pi^2} \cdot \frac{1}{D}}$$

wobei D der Trommeldurchmesser und g die Gravitationskonstante ist.

Die Umdrehungsgeschwindigkeiten der Kugelmühle betragen vorzugsweise 20 % bis 95 %, weiter vorzugsweise 50 % bis 90 % und besonders bevorzugt 55 % bis 86 % der kritischen Drehzahl $n_{krit}$.

Die Umdrehungsgeschwindigkeiten dürfen nicht zu hoch sein, um eine langsame Verformung der Metallpartikel zu begünstigen. Andererseits benötigt der Kupfer- oder Messinggrieß (Goldbronzegrieß) - etwa im Gegensatz zu Aluminiumgrieß - aufgrund der geringeren Duktilität des Messings (bzw. Kupfers) einen eher höheren Energieeintrag und daher höhere Umdrehungsgeschwindigkeiten. Um eine langsame Verformung zu bewirken, werden bei dem erfindungsgemäßen Verfahren bevorzugt auch leichte Mahlkugeln verwendet.

Im Unterschied zu herkömmlichen Mahlverfahren wird der Kupfer- oder Messinggrieß bei dem erfindungsgemäßen Verfahren zum überwiegenden Anteil nicht vermahlen bzw. zerkleinert, sondern äußerst schonend über einen längeren Zeitraum verformt.

Die oben angeführten Bedingungen führen zu einer sehr schonenden Mahlung, bei der der Metallgrieß langsam ausgeformt wird und Brüche der Metallpartikel infolge eines Kugelstoßes mit hoher kinetischer Energie vermieden werden.

Das Mahlgut wird abfiltriert und der erhaltene Filterkuchen in einer weiteren Kugelmühle mit kugelförmigen Mahlkörpern, Lösungsmittel und Mahladditiv vermahlen.

Das Mahlgut wird durch Spülen mit Lösungsmittel von den Mahlkugeln getrennt und anschließend aufkonzentriert.

In einem weiteren, bevorzugten Verfahrensschritt können die erhaltenen Metalleffektpigmente einer Größenklassifikation unterzogen werden. Diese Klassifikation sollte schonend durchgeführt werden, um die dünnen Metallpigmente nicht zu zerstören. Es kann sich dabei beispielsweise um eine Nasssiebung, eine Dekantierung oder auch um eine Trennung durch Sedimentation (infolge der Schwerkraft oder durch Zentrifugieren) handeln. Bei der Nasssiebung wird i. d. R. der Grobanteil herausgesiebt. Anschließend wird die Suspension von überschüssigem Lösemittel getrennt (z.B. mit Hilfe einer Filterpresse, Zentrifuge oder Filter).

In einem weiteren bevorzugten Verfahrensschritt können die erfindungsgemäßen kupferhaltigen Metalleffektpigmente einer sogenannten Feuerfärbung unterzogen werden. Bei dieser Pigmentbehandlung wirkt Luftsauerstoff über einen bestimmten Zeitraum unter definierter Temperatur auf das kupferhaltige Metalleffektpigment ein, wodurch sich eine dünne Oxidschicht auf dem Metallplättchen ausbildet. Durch Interferenzreflexion werden interessante Farbnuancierungen hervorgerufen. Feuereingefärbte kupferhaltige Metalleffektpigmente werden u. a. in den Farbtönen Englischgrün-, Zitron-, Dukatengold- und Feuerrottönen kommerziell gehandelt.

Vorteilhaft können die erfindungsgemäßen kupferhaltigen Metalleffektpigmente als pastenförmige Produkte in allen bekannten Farbtönen bereitgestellt werden.

Der Festkörpergehalt der erfindungsgemäße Metalleffektpigmente enthaltenden Pasten beträgt 30 bis 90 Gew.-%, bevorzugt 40 bis 75 Gew.-% und besonders bevorzugt 45 bis 70 Gew.-%, bezogen auf die Paste.

Die durch Vermahlung hergestellten sehr dünnen kupferhaltigen Metalleffektpigmente unterscheiden sich von durch PVD-Verfahren zugänglichen Goldbronzepigmenten, wie sie beispielsweise in der EP 1 529 084 B1 beschrieben sind.

Zum einen weisen die erfindungsgemäßen kupferhaltigen Metalleffektpigmente im Fall von Goldbronzen überraschenderweise eine weitgehend homogene Zusammensetzung hinsichtlich ihrer beiden Legierungsbestandteile Kupfer und Zink auf. Bei den durch PVD-Verfahren hergestellten Pigmenten stellte sich heraus, dass oftmals eine Phasenseparation der beiden Metalle stattfindet.

Ein anderer struktureller Unterschied besteht darin, dass die Oberflächen der erfindungsgemäßen kupferhaltigen Metalleffektpigmente natürlicherweise eine höhere Mikrorauheit als jene durch PVD-Verfahren erzeugten Pigmente aufweisen. Diese erhöhte Mikrorauheit ist auf die Einwirkung der Mahlkörper auf die Pigmente zurückzuführen.

Ein weiterer struktureller Unterschied zwischen PVD-Pigmenten und den erfindungsgemäßen Pigmenten ist im Randbereich der Pigmente zu erkennen.

PVD-Pigmente werden durch Aufdampfung auf ein Trägermaterial und nachfolgende Ablösung und Zerkleinerung hergestellt. Hierdurch bedingt weisen PVD-Pigmente in der Regel gerade Bruchkanten auf. Ferner nimmt die PVD-Pigmentdicke von der Mitte des Pigmentes zum Randbereich nicht ab.

Bei den erfindungsgemäßen kupferhaltigen Effektpigmenten erfolgt in der Regel eine Abflachung der Pigmentdicke zum Randbereich. Ferner weist der Randbereich in der Regel keine geraden Kanten, sondern einen unregelmäßig geformten Rand auf.

Die strukturellen, auf die verschiedenen Herstellungsverfahren zurückzuführenden, Unterschiede zwischen PVD-Pigmenten und den erfindungsgemäßen Pigmenten sind in REM-Aufnahmen gut erkennbar.

Besonders vorteilhaft werden die erfindungsgemäßen Metalleffektpigmente durch Austrocknen in eine Pulverform, vorzugsweise in eine nichtstaubende Pulverform, beispielsweise in ein stabilisiertes Metallpulver, überführt. Das getrocknete Pulver kann durch Zugabe sehr kleiner Mengen Lösemittel (< 10%) in einem geeigneten Homogenisator zu einem nicht staubenden Metallpulver weiterverarbeitet werden. Auch kann der Filterkuchen zunächst ausgetrocknet und anschließend mit einem anderen Lösemittel wieder angepastet werden (Umnetzen).

Überraschenderweise können die erfindungsgemäßen Metalleffektpigmente aber auch durch Versetzen des Filterkuchens mit einer geeigneten Dispersion eines geeigneten Harzes zu einem Granulat, Pellet, Brikett, Tabletten oder Würstchen überführt werden. Diese Angebotsformen besitzen die Vorteile, dass sie nicht stauben, eine leichte Dosierbarkeit aufweisen und hervorragend dispergierbar sind.

Die Pelletierung kann auf einem Pelletierteller auf herkömmliche Art und Weise durchgeführt werden. Das Tablettieren kann in einer Tablettiervorrichtung erfolgen. Die Würstchen können durch ein Pressverfahren aus Pigmentpaste oder -pulver hergestellt werden oder indem diese durch einen Extruder extrudiert wird und die extrudierten Pastenstränge durch eine umlaufende Messeranordnung zerteilt werden. Ein Granulieren der erfindungsgemäßen kupferhaltigen Metalleffektpigmente (insbesondere Goldbronzepigmente) kann beispielsweise durch Sprühgranulieren erfolgen.

Die erfindungsgemäßen kupferhaltigen Metalleffektpigmente können äußerst vorteilhaft in Granulaten oder Pellets mit hohen Pigmentgehalten, beispielsweise von 90 Gew.-% bis 35 Gew.-%, vorzugsweise 70 Gew.-% bis 40 Gew.-%, jeweils bezogen auf das Gesamtgewicht an Granulat oder Pellet bereitgestellt werden.

Aufgrund der hohen spezifischen Oberfläche der erfindungsgemäßen Pigmente werden zu deren Pelletierung vorzugsweise relativ große Mengen an Dispergierharz verwendet. Vorzugsweise werden 2 bis 50 Gew.-%, weiter bevorzugt 5 bis 30 Gew.-%, Harz, bezogen auf die Gesamtformulierung der Pellets, verwendet.

Zur Pelletierung kann eine Vielzahl von Dispergierharzen verwendet werden. Beispiele hierfür sind sowohl natürlich vorkommende wie auch synthetische Harze. Sie umfassen beispielsweise Alkydharze, Carboxymethyl- und Carboxyethylcelluloseharze, Cellulose Acetat, Cellulose Acetat Propionat (CAP) und Cellulose Acetat Butyrat (CAB), Cumarol-Indenharze, Epoxidester, Epoxid-Melamin und Epoxid-Phenol-Kondensate, Ethyl- und Methylcellulose, Ethylhydroxyethylcellulose, Hydroxyethylcellulose, Hydroxypropylmethylcellulose, Keton- und Maleinsäureharze, Kolophoniumharze, Melaminharze, Nitrocelluloseharze, Phenol- und modifizierte Phenolharze, Polyacrylamid-, Polycarbonat-, Polyamid-, Polyester-, Polyether-, Polyurethan-, und Vinylharze.

Unter diesen polymeren Harzen sind insbesondere zu erwähnen: acrylatische Copolymere und Acrylesterharze, Polyacrylonitril- und Acrylonitrilcopolymerharze, Copolymere aus Butadien und Vinylidenchloride, Butadien/Styrol Copolymere, Methylacrylat- und Methylmethacrylatcopolymere sowie Polybuten-, Polyisobutylen-, Polyvinylacetat-, Polyvinylakohol-, Polyvinylchlorid-, Polyvinylether-, Polyvinylpyrrolidon- und Polystyrolharze. Weitere Copolymere beinhalten Styrol/Maleinsäureanhydrid- und Styrol/Schellackharze, Vinylchlorid/Vinylacetat-, Vinylchlorid/Vinylether- und Vinylchlorid/Vinylidenchloridharze.

Ferner kommen natürlich vorkommende Harze wie Gummi Arabicum, Gutta Percha, Casein und Gelatine in Betracht.

Bevorzugt sind Aldehydharze, wie die Laropalserie der BASF AG, Ludwigshafen. Weiterhin kommen Wachse als Bindermaterialien in Frage. Hier sind als Beispiele natürliche Wachse wie Bienenwachs, Candelilla-, Carnauba-, Montan- sowie Parafinwachse zu nennen. Ebenso kommen synthetische Wachse wie beispielsweise PE-Wachse in Betracht.

Die vorgenannten Präparationen lassen sich beispielsweise in Lacksysteme oder Druckfarben sehr gut einarbeiten, ohne dass es zu unerwünschten Agglomerationen von den Pigmenten kommt.

Die erfindungsgemäßen kupferhaltigen Metalleffektpigmente mit der typischen Coloristik von Kupfer oder Messing können in Beschichtungszusammensetzungen wie Coatings, Anstrichen, Lacken, Druckfarben, Pulverlacken, Kunststoffen, Wertschriften- und Sicherheitsdrucken, Keramiken und kosmetischen Formulierungen Verwendung finden.

Die erfindungsgemäßen Kupfer- oder Messingpigmente können besonders vorteilhaft in Druckfarben für den Tief-, Flexo- oder Siebdruck eingesetzt werden. Besonders bevorzugt ist der Einsatz dieser Pigmente in Tief-, Sieb- und Flexodruckfarben zur Herstellung von Folienkonterapplikationen mit spiegelartigem Effekt.

Ein weiterer Gegenstand der Erfindung ist eine Beschichtungszusammensetzung, vorzugsweise eine Druckfarbe, bevorzugt eine Tief-, Flexo- oder Siebdruckfarbe, die die erfindungsgemäßen kupferhaltigen Metalleffektpigmente enthält.

Druckfarben enthalten Lösemittel bzw. Lösemittelgemische. Diese dienen u. a. zum Lösen der Bindemittel, aber auch zur Einstellung wichtiger Anwendungseigenschaften dieser Druckfarben, wie beispielsweise der Viskosität oder der Trocknungsgeschwindigkeit. Für Tief- und Flexodruckfarben eingesetzte Lösemittel umfassen insbesondere niedrig siedende Lösemittel. Der Siedepunkt beträgt im Regelfall nicht mehr als 140°C. Höher siedende Lösemittel werden nur in kleineren Mengen zur Einstellung der Trocknungsgeschwindigkeit eingesetzt.

Beispiele geeigneter Lösemittel für Flüssigdruckfarben umfassen Ethanol, 1-Propanol oder 2-Propanol, substituierte Alkohole, beispielsweise Ethoxypropanol oder Methoxypropanol oder Ester, beispielsweise Ethylacetat, Isopropylacetat, n-Propyl- oder n-Butylacetat. Es können selbstverständlich auch Gemische verschiedener Lösemittel eingesetzt werden. Beispielsweise kann es sich um ein Gemisch aus Ethanol und Estern wie Ethylacetat oder n- Propylacetat handeln.

Für das Drucken mit Tief- und Flexodruckfarben ist es regelmäßig empfehlenswert, dass der Anteil der Ester am Gesamtlösemittel ca. 20 bis 25 Gew.-% nicht überschreitet. Als Lösemittel für Flüssigdruckfarben sind auch Wasser oder überwiegend wässrige Lösemittelgemische einsetzbar. Je nach Art der Druckfarbe werden üblicherweise 10 bis 80 Gew.-% Lösemittel bezüglich der Summe aller Bestandteile eingesetzt.

Für erfindungsgemäße Druckfarben erweist sich jedoch ein Bereich von 60 bis 80 Gew.-% Lösemittel als besonders vorteilhaft.

Strahlungshärtbare Druckfarben enthalten im Allgemeinen nicht die oben genannten Lösemittel, sondern Reaktiwerdünner. Reaktiwerdünner erfüllen typischerweise eine Doppelfunktion. Einerseits dienen sie zum Vernetzen bzw. Härten der Druckfarbe, andererseits dienen sie aber auch wie konventionelle Lösemittel (DE 20 2004 005 921 U1) zum Einstellen der Viskosität. Beispiele umfassen Butylacrylat, 2-Ethylhexylacrylat, sowie insbesondere mehrfunktionelle Acrylate wie 1,4-Butandioldi(meth)acrylat, 1,6-Hexandioldi(meth)acrylat oder Trimethylolpropantri(meth)acrylat.

Als Bindemittel für die erfindungsgemäßen Druckfarben können prinzipiell die für Flüssig-Druckfarben üblichen Bindemittel eingesetzt werden. Je nach dem gewünschten Anwendungszweck und den gewünschten Eigenschaften trifft der Fachmann eine geeignete Auswahl. Beispiele geeigneter Bindemittel umfassen Polyester, Polyamide, PVC-Copolymerisate, aliphatische und aromatische Ketonharze, Melamin-Harnstoff-Harze, Melamin-Formaldehyd-Harze, Maleinate, Kolophoniumderivate, Polyvinylbutyrale, Casein bzw. Casein-Derivate, Ethylcellulose, Nitrocellulose oder aromatische bzw. aliphatische Polyurethane. Es können auch Polymere oder Copolymere von Vinylacetat, Vinylalkohol, Acrylaten, Methacrylaten, Vinylpyrrolidon oder Vinylacetalen eingesetzt werden. Von besonderem Vorteil können funktionelle Gruppen aufweisende hyperverzweigte Polymere, beispielsweise hyperverzweigte Polyurethane, Polyharnstoffe oder Polyesteramide eingesetzt werden, wie von WO 02/36695 und WO 02/36697 offenbart. Es können selbstverständlich auch Gemische verschiedener polymerer Bindemittel eingesetzt werden, vorausgesetzt, die ausgewählten Bindemittel weisen in Kombination miteinander keine unerwünschten Eigenschaften auf. Die Menge aller Bindemittel beträgt üblicherweise 2 bis 40 Gew.-% bzgl. der Summe aller Bestandteile der Druckfarbe.

Besonders bevorzugte Bindemittel umfassen beispielsweise Nitrocellulose, Ethylcellulose, Hydroxyethylcellulose, Acrylate, Polyvinylbutyrale sowie aliphatische und aromatische Polyurethane und Polyharnstoffe, insbesondere hyperverzweigte Polyurethane und Polyharnstoffe sowie Mischungen davon.

Als Bindemittel für wasserverdünnbare Druckfarben kommen insbesondere Copolymere auf Basis von (Meth)acrylsäure und/oder deren Estern mit Styrol in Frage. Derartige Bindemittel sind als Lösungen oder Dispersionen für den Einsatz in Druckfarben beispielsweise unter dem Namen Zinpol® (Fa. Worlee) kommerziell erhältlich. Weitere Beispiele umfassen aromatische bzw. aliphatische wässrige Polyurethane, Polyester und wässrige Polyamide.

Für Offsetdruckfarben bevorzugte Bindemittel umfassen beispielsweise Kolophoniumharze oder modifizierte Kolophoniumharze. Beispiele für modifizierte Kolophoniumharze umfassen mit Polyolen wie beispielsweise Glycerin oder Pentaerythrit ganz oder teilweise veresterte Kolophoniumharze.

Strahlungshärtbare Druckfarben umfassen Bindemittel, die vernetzbare Gruppen umfassen, wie beispielsweise olefinische Gruppen, Vinylether- oder Epoxidgruppen. Hier liegt die Summe der Bindemittel (inklusive Reaktiwerdünner) in der Regel in einem Bereich von 30 bis 90 Gew.-% aller Bestandteile der Druckfarbe.

Die erfindungsgemäßen Druckfarben können weiterhin einen oder mehrere Hilfsstoffe beziehungsweise Additive umfassen. Beispiele für Additive und Hilfsstoffe sind Füllstoffe wie Calciumcarbonat, Aluminiumoxidhydrat oder Aluminium- bzw. Magnesiumsilikat. Wachse erhöhen die Abriebfestigkeit und dienen der Erhöhung der Gleitfähigkeit. Beispiele sind insbesondere Polyethylenwachse, oxidierte Polyethylenwachse, Petroleumwachse oder Ceresinwachse. Fettsäureamide können zur Erhöhung der Oberflächenglätte eingesetzt werden. Weichmacher dienen der Erhöhung der Elastizität des getrockneten Films. Für strahlungshärtbare Druckfarben wird als Additiv weiterhin mindestens ein Fotoinitiator oder ein Fotoinitiatorsystem eingesetzt. Zum Dispergieren der Effektpigmente können Dispergierhilfsmittel eingesetzt werden. Mittels Fettsäuren kann ein Aufschwimmen der Effektpigmente in der gedruckten Schicht erreicht werden, so dass die Pigmente in an der oberen Grenzfläche der Druckschicht angereichert sind. Hierdurch können vorteilhaft verbesserte Metallic-Effekte erzielt werden. Weiterhin können auch Antiabsetzmittel zugesetzt werden. Derartige Zusätze verhindern die Sedimentation der Effektpigmente. Beispiele umfassen Kieselsäure, Cellulose-Derivate oder auch Wachse.

Zur Formulierung der besonders bevorzugten dünnflüssigen Tief- oder Flexodruckfarben ist der Zusatz von Antiabsetzmitteln meist empfehlenswert, obwohl nicht immer unbedingt erforderlich. Die Gesamtmenge aller Additive und Hilfsstoffe sollte üblicherweise 20 Gew.-%, bezüglich der Summe aller Bestandteile der Druckfarbe, nicht übersteigen und beträgt bevorzugt 0,1 bis 10 Gew.-%.

Die Herstellung der erfindungsgemäßen Druckfarben kann in prinzipiell bekannter Art und Weise durch intensives Vermischen bzw. Dispergieren der Bestandteile in üblichen Apparaturen, beispielsweise Dissolvern oder Rührwerken, erfolgen. Der Fachmann wird beim Einsatz von Dissolvern darauf achten, dass der Energieeintrag nicht zu hoch ist, um ein Beschädigen der erfindungsgemäßen Metalleffektpigmente zu vermeiden. Umgekehrt muss der Energieeintrag natürlich so hoch sein, um ein ordnungsgemäßes Dispergieren der Pigmente zu ermöglichen. Falls neben den erfindungsgemäßen Metalleffektpigmenten noch übliche Farbpigmente eingesetzt werden, kann es empfehlenswert sein, diese in einem Teil oder in der Gesamtmenge des Lösemittels, Bindemittels sowie gegebenenfalls der Hilfsstoffe der Druckfarbe vorzudispergieren, und die erfindungsgemäßen Pigmente erst später zuzugeben. Auf diese Art und Weise wird eine besonders gute Dispergierung der zusätzlichen Pigmente erreicht, ohne die Pigmente durch zu starke Dispergierung zu schädigen. Anstelle der Pigmente können auch vordispergierte Pigmentkonzentrate zugegeben werden. Ganz besonders elegant kann hierbei auch eine handelsübliche Druckfarbe in geringen Mengen eingesetzt werden, vorausgesetzt, die zugesetzte Druckfarbe ist mit der Rezeptur der Druckfarbe verträglich und verschlechtert nicht deren Eigenschaften.

Ein mit erfindungsgemäßen Metalleffektpigmenten beschichteter Gegenstand kann ein Gebrauchsgegenstand, wie beispielsweise ein Druckerzeugnis oder ein Kraftfahrzeug, aber auch ein für sonstige (kommerzielle) Zwecke einsetzbarer Gegenstand aus Papier, Pappe, Karton, Kunststoff, Folie, Metall, Glas, Stein oder anderen bekannten Werkstoffen, sein.

Die nachfolgend aufgeführten Beispiele erläutern die Erfindung ohne sie jedoch zu beschränken:

**Beispiel 1:**

**a) Verdüsung Metallgrieß:**

Zur Herstellung von erfindungsgemäßen Messingpigmenten wurden in einem Induktionsofen 70 Gew.-% Kupfer und 30 Gew.-% Zink eingebracht und aufgeschmolzen. Anschließend wurde die Messingschmelze in einen Induktionsrinnenofen mit Vorherd übergeben. Die im Vorherd bei einer Temperatur von etwa 1050°C flüssig vorliegende Messingschmelze wurde durch eine am Vorherd angebrachte Zerstäubungsdüse vertikal nach unten verdüst. Zum Zerstäuben der Messingschmelze wurde eine geschlossene Düse eingesetzt. Die bei der Verdüsung entstandenen Messingpartikel erstarren und erkalten im Fluge. Die Verdüsung erfolgte unter Zufuhr von Heißluft bei etwa 400 °C. Das zur Verdüsung eingesetzte Heißgas wurde verdichtet, danach in Gaserhitzern erhitzt und anschließend in die zu zerstäubende Messingschmelze eingetragen. Die Abscheidung der Messingpartikel erfolgte mittels Zentrifugalkraft. Der dort abgeschiedene pulverförmige Messinggrieß hatte einen $d_{50}$ von < 60 $\mu$m. Die Gas-Feststoff-Trennung erfolgte in einem Filter. Die weitere Auftrennung dieses Messinggrießes erfolgte durch weitere Klassierschritte. Daraus resultierte ein pulverförmiger Messingfeinstgrieß ("Messing 70:30 Reichgoid"), mit einem $d_{Grieß,10}$ von 1,4 $\mu$m, einem $d_{Grieß,50}$ von 2,4 $\mu$m und einem $d_{90}$ von 4,0 $\mu$m sowie einen $d_{Grieß,98}$ von 6 $\mu$m hergestellt.

b) **Herstellung Vermahlungsadditiv** (in Anlehnung an die EP 1 304 210 A1): 50 g Pripol 1009 (hydrierte C36-Dimersäure von Unichema) und 89 g MPEG 750 (Methoxypolyethylenglycol) wurden in ein Glasreaktionsgefäß eingewogen und unter $N_2$-Schutzgas und Rühren auf 80 °C erwärmt. Anschließend wurde 0,8 g p-Toluolsulfonsäure (Katalysator) hinzugegeben und auf 180 °C aufgeheizt. Entstehendes Reaktionswasser wurde über einen Wasserabscheider abgeschieden. Anhand der Säurezahl wurde der Fortschritt der Reaktion kontrolliert. Die Säurezahl wurde gemäß der DIN 53402 bestimmt. Die Reaktion wurde nach Erreichen einer Säurezahl von etwa 24 mg KOH/g Additiv gestoppt. Dies entspricht einem Veresterungsgrad von ca. 67 %. Das mittlere Molekulargewicht des entstandenen Esters betrug ca. 1.750 g/ mol.

**c) Vermahlung:**

Zur Nassvermahlung des gemäß Schritt a) hergestellten Messingfeinstgrießes wurden 400 g dieses Metallgrießes in eine Mühle (Länge: 32 cm, Breite: 19 cm) mit 10 kg Chromstahlkugeln (Durchmesser: 3 mm) ur d 900 g Isopropylacetat sowie 30 g Mahladditiv nach Bsp. 1 b eingebracht und bei 80 U/min 30 h lang vermahlen. Das Mahlprodukt wurde durch Spülen mit Lösemittel von den Mahlkugeln getrennt und abfiltriert. Der Filterkuchen wurde anschließend in eine zweite Mühle eingebracht. Die in diese Mühle mit einer Menge von 400 g eingebrachte Messingpaste wurde mit Hilfe von 10 kg Chromstahlkugeln (Durchmesser: 1,3 mm) mit einer Drehzahl von 60 U/min 30 h lang mit ca. 900 g Isopropylacetat und etwa 25 g Mahladditiv nach Bsp. 1 b vermahlen. Anschließend wurde die Messingpigmentpaste durch Spülen mit Lösemittel von den Mahlkugeln getrennt und danach auf einen Feststoffgehalt von 70 Gew.-% aufkonzentriert.

**Beispiel 2:**

Analog Beispiel 1, jedoch wurde bei der Vermahlung anstelle von Isopropylacetat als Lösemittel N-Propylacetat eingesetzt.

**Beispiel 3:**

a) Verdüsung Kupfergrieß

Zur Herstellung von erfindungsgemäßen Kupferpigmenten wurde in einem Induktionsofen Kupfer chargenmäßig eingebracht und aufgeschmolzen. Anschließend wurde die Kupferschmelze in einen Induktionsrinnenofen mit Vorherd übergeben. Die im Vorherd vorliegende Kupferschmelze wurde durch eine Zerstäubungsdüse vertikal nach unten verdüst. Die bei der Verdüsung entstandenen Kupferpartikel erstarrten und erkalteten im Fluge. Die Verdüsung erfolgte unter Zufuhr von Heißluft bei etwa 500°C. Die Abscheidung der Kupferpartikel erfolgte mittels Zentrifugalkraft. Der dort abgeschiedene pulverförmige Kupfergrieß hatte einen $d_{Grieß,50}$ von < 60 $\mu$m. Die Gas-Feststoff-Trennung erfolgte in einem Filter. Aus der vorliegenden Kupfergrießfraktion wurde durch weitere Klassierschritte ein Kupferfeinstgrieß mit einer Partikelgröße $d_{Grieß,50}$ von < 10 $\mu$m und aus diesem ein Kupferfeinstgrieß mit einem $d_{Grieß,10}$ von 1,2 $\mu$m, einem $d_{Grieß,50}$ von 3 $\mu$m und einem $d_{Grieß,90}$ von 4,4 $\mu$m sowie einen $d_{Grieß,98}$ von 7 $\mu$m hergestellt

b) Vermahlung:

Die Nassvermahlung des gemäß Schritt 3a) hergestellten Kupferfeinstgrießes wurde analog Beispiel 1c durchgeführt.

**Vergl.-Beispiel 4:**

Die Herstellung von kommerziell gehandeltem Goldbronzepigmentpulver für Tief- und Flexodruckfarben ("ROTOFLEX"der Fa. Eckart GmbH) erfolgt im bekannten mehrstufigen Trockenmahlverfahren (Hametag-Verfahren) mit Stearinsäure als Vermahlungshilfsmittel. Als Ausgangsmaterial wurde ein Messinggrieß mit 70 Gew.% Kupfer und 30 Gew.% Zink mit einem mittleren Teilchendurchmesser $d_{Grieß,50}$ von 140 $\mu$m eingesetzt. Aus den als klassiertes Mahlgut vorliegenden leafing Goldbronzepigmenten mit einem mittleren Teilchendurchmesser $d_{50}$ = 8$\mu$m wurde mittels einer nachfolgenden Oberflächenmodifizierung mit 2,5% Zitronensäure ein Goldbronzepigment mit non-leafing Eigenschaften hergestellt.

**Vergl.-Beispiel 5:**

Kommerziell erhältliches PVD-Pigment Metalure A (Fa. Eckart GmbH, Deutschland). Dieses Pigment ist in der kommerziell erhältlichen Druckfarbe ("ULTRASTAR" Fa. Eckart) für den Tief- und Flexodruck vorhanden.
Diese Druckfarbe enthält weiterhin einen gelben und einen orangen Tonerfarbstoff, um einen goldfarbenen Eindruck hervorzurufen.
Zur Bestimmung der Teilchendicken wurden Proben des erfindungsgemäßen Beispiels 1 und der Vergleichsbeispiele 4 und 5 mittels eines Feldionen-Rasterelektronenmikroskops charakterisiert.
Für die Bestimmung der Dickenverteilung mittels REM wurden die Proben folgendermaßen präpariert:
Die aus nassvermahlenem Messinggrieß hergestellten, als Paste oder Filterkuchen vorliegenden, plättchenförmigen Messingpigmente, wurden mit Aceton gewaschen und anschließend getrocknet.
Ein in der Elektronenmikroskopie übliches Harz, beispielsweise TEMPFIX (Gerhard Neubauer Chemikalien, D-48031 Münster, Deutschland), wurde auf einen Probenteller aufgebracht und auf einer Heizplatte bis zum Erweichen erhitzt. Nachfolgend wurde der Probenteller von der Heizplatte genommen und das Messingpulver auf das erweichte Harz gestreut. Das Harz wurde durch die Abkühlung wieder fest und die aufgestreuten Messingpigmente konnten - bedingt durch das Wechselspiel zwischen Adhäsion und Schwerkraft - nahezu senkrecht stehend und fixiert auf dem Probenteller präpariert werden. Dadurch sind die Messingpigmente im Elektronenmikroskop seitlich gut zu vermessen.

Bei der Vermessung der Dicke wurde der azimuthale Winkel des Pigmentes zu einer zur Oberfläche normalen Ebene geschätzt und bei der Dickenauswertung nach der Formel

$$h_{eff} = h_{mess} / \cos \alpha$$

berücksichtigt.

Von den errechneten $h_{eff}$-Werten wurden anhand der relativen Häufigkeiten die Summendurchgangsverteilungskurve erstellt. Es werden jeweils 50 bis 100 Teilchen ausgewählt.

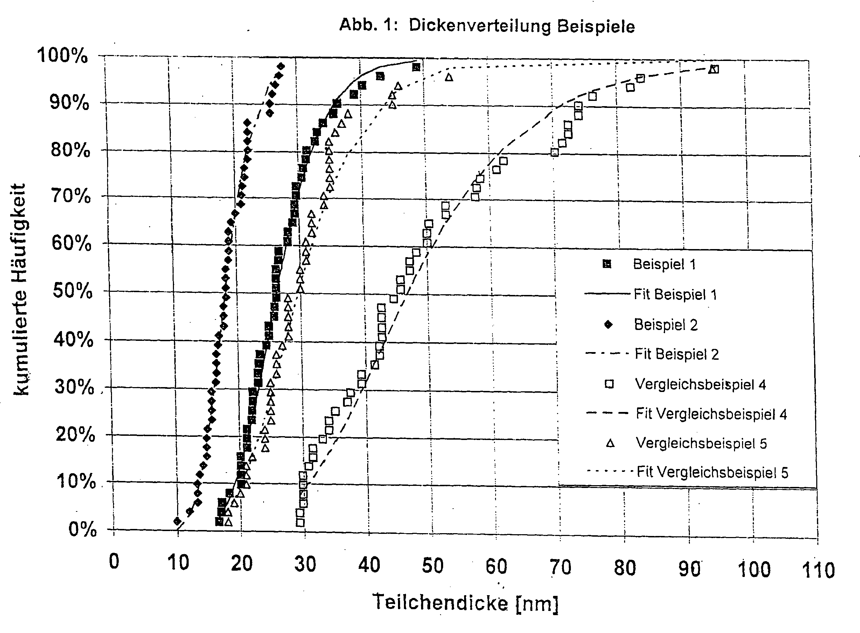

Die Summendurchgangsverteilungen der Dickenverteilung der Proben des erfindungsgemäßen Beispiels 1 und der Vergleichsbeispiele 4 und 5 sind in **Abb. 1** dargestellt. Die Messwerte wurden jeweils mit einer log-Normalfunktion gefittet. Deutlich sind die viel geringeren Teilchendicken der gesamten Teilchenverteilungen der Proben des erfindungsgemäßen Beispiels 1 im Vergleich zu den Proben der Vergleichsbeispiele 4 und 5 zu erkennen.

Aus der nachfolgenden **Tab. 1** ist die physikalische Charakteristik der erfindungsgemäßen Messingpigmente (Beispiel 1) im Vergleich zu kommerziell gehandeltem Goldbronzepigmentpulver (Vergleichsbeispiel 4) der Fa. Eckart und dem PVD-Aluminiumpigment (Vergleichsbeispiel 5) der Fa. Eckart anhand der $d_{10}$-, $d_{50}$- und $d_{90}$-Werte und die Kennwerte $h_{10}$, $h_{50}$ und $h_{90}$ und daraus berechneten Span-Werte der Dickenmessung aus den REM-Untersuchungen zu entnehmen. Die $h_{10}$, $h_{50}$ und $h_{90}$-Werte wurden mit Hilfe der Quantilfunktion aus den Originaldaten der Dickenauszählung berechnet.

Die Längsausdehnung d der Pigmente wurde mit Hilfe eines Lasergranulometers (Cilas 1064, Firma Cilas, Frankreich) bestimmt und als Maß der mittleren Längsausdehnung wie üblich der $d_{50}$-Wert der Summendurchgangsverteilung in $\mu$m gewählt.

**Tab. 1: Physikalische Charakterisierung Pigmente**

| Proben | Größen | | | Dicken | | | | Formfaktor |
|---|---|---|---|---|---|---|---|---|
| | $d_{10}$ [$\mu$m] | $d_{50}$ [$\mu$m] | $d_{90}$ [$\mu$m] | $h_{10}$ [nm] | $h_{50}$ [nm] | $h_{50}$ [nm] | Span Dickenverteilung [%] | |
| Beispiel 1 | 3,5 | 8,3 | 13 | 20,2 | 26,2 | 35,8 | 0,6 | 317 |
| Beispiel 2 | 2,9 | 8,2 | 12,6 | 13,6 | 18,3 | 25,8 | 0,67 | 448 |
| Vergleichsbeispiel 4 | 3,3 | 8 | 15 | 29,9 | 45,2 | 74,1 | 1 | 177 |
| Vergleichsbeispiel 5 | 3,5 | 10 | 17,5 | 21 | 29 | 38,7 | 0,6 | 476 |

Aus den Werten der **Tab. 1** ist zu entnehmen, dass die erfindungsgemäßen nonleafing Messingpigmente gemäß Beispiel 1 und 2 sowohl eine geringere mittlere Dicke $h_{50}$ als auch einen geringeren $h_{90}$-Wert als die stabilisierten leafing Goldbronzepigmente "ROTOFLEX" der Fa. Eckart GmbH, D-90763 Fürth gemäß Vergleichsbeispiel 4 aufweisen. Überraschenderweise weisen sie sogar geringere Pigmentdicken auf als die PVD-Aluminiumpigmente "Metalure A" der Fa. Eckart gemäß Vergleichsbeispiel 5.

Der Span der Dickenverteilung ist bei den erfindungsgemäßen Pigmenten vergleichbar mit den PVD-Aluminiumpigmenten. Dies war bisher nicht aus einer Naßvermahlung zugänglich. Das konventionelle Goldbronzepigment aus der Naßmahlung (Vergleichsbeispiel 4) zeigt einen deutlich höheren Span.

Weiterhin ist aus **Tab. 1** ersichtlich, dass die erfindungsgemäßen Messingpigmente gemäß Beispiel 1 eine wesentlich engere Dickenverteilung (Span) als die konventionellen Goldbronzepigmente des Vergleichsbeispiels 4 aufweisen. Zudem weisen die erfindungsgemäßen Messingpigmente gemäß Beispiel 1 und 2 eine geringere Größe $d_{90}$ als die Pigmente der Vergleichsbeispiele 4 und 5 auf.

Die Summendurchgangsverteilungen der Dickenverteilung der erfindungsgemäßen Beispiele 1 und 2 und der Vergleichsbeispiele 4 und 5 sind in **Abb. 1** dargestellt. Deutlich sind die viel geringeren Teilchendicken der gesamten Teilchenverteilungen der erfindungsgemäßen Beispiele 1 und 2 im Vergleich zu den kommerziell erhältlichen Goldbronzepigmenten gemäß Vergleichsbeispiel 4 und den kommerziell erhältlichen PVD-Aluminiumpigmenten gemäß Beispiel 5 zu erkennen. Die Messwerte wurden jeweils mit einer log-Normalfunktion gefittet.

Zur weiteren Charakterisierung der erfindungsgemäßen Messingpigmente wurden sogenannte Konterapplikationen auf transparenten Folien erstellt. Hierzu wurde eine MELINEX 400 Folie (PET-Folie, 50 $\mu$m) mit einer Tiefdruckfarbe auf Basis eines kommerziell gehandelten Polyvinylbutyrals (PVB) und einer Mischung aus Methoxypropanol und Ethylacetat mittels einer Druckmaschine bedruckt.

Die pigmentierten Folienkonterapplikationen wurden optisch durch eine Glanzmessung bei 60° in Anlehnung an DIN 67 530 (Gerät: micro-TRI-gloss von Byk-Gardner, D-82538 Geretsried, Deutschland) charakterisiert. Kalibriert wurde mittels Dunkelkalibrierung sowie einer schwarzen Spiegelglasplatte mit Werten von 92 für 60°.

Die Farbdichte wurde mit einem Densitometer (Gerät: Densitometer, Fa. X-Rite , D-63263 Neu-Isenburg) gemessen. Kalibriert wurde mit Hilfe eines Weißstandards und des unbedruckten Substrats bei einer Wellenlänge im gelben Bereich.

Die Definition der Farbdichte von Druckmustern ist folgendermaßen:

*Farbdichte = - lg Remission*

Dabei werden die betrachteten Oberflächen in der Aufsicht vermessen.

Die auf Grundlage von Druckmaschinenandrucken (Druckmaschine: Rotova 300, Fa. Rotocolor, 3 Farbwerke; Druckgeschwindigkeit 75 m/min, Viskosität 15 s DIN-4-Auslaufbecher, 60, 70, 80 und 90 Linien/cm; Pigmentierungshöhe 25%) ermittelten optischen Eigenschaften von mit erfindungsgemäßen Messingpigmenten gemäß Beispiel 1 und konventionellen Goldbronzepigmenten gemäß Vergleichsbeispiel 4 sowie eingefärbten, herkömmlichen PVD-Aluminiumpigmenten gemäß Vergleichsbeispiel 5 pigmentierten Folienkonterapplikationen sind in nachfolgender **Tab. 2** dargestellt.

Dabei wurde bei Vergleichsbeispiel 5 die Druckfarbe ULTRASTAR (Fa. Eckart) mit zwei unterschiedlichen Konzentrationen einer Mischung von gelben (Yellow 79) und orangen (Solvent Orange 41) Farbstoffen verwendet (Beispiel 5a und 5b). Die Farbstoffe wurden in Form der UltraStar Toner-Reihe (UltraStar Toner TY-21 und TO-11; Fa. Eckart) vermischt, wobei es sich bei der Tonerreihe jeweils um Dispersionen der Farbstoffe in Methoxypropanol handelte.

**Tab. 2: Optische Charakterisierung Pigmente I**

| Probe | Glanz (60°) | | | | Farbdichte | | | |
|---|---|---|---|---|---|---|---|---|
| Linien/cm | 60 l/cm | 70 l/cm | 80 l/cm | 90 l/cm | 60 l/cm | 70 l/cm | 80 l/cm | 90 l/cm |
| Beispiel 1 | 500 | 488 | 440 | 428 | 1,45 | 1,44 | 1,32 | 1,22 |
| Vergleichs-beispiel 4 | 243 | 246 | 232 | 230 | 1,00 | 1,00 | 0,84 | 0,77 |
| Vergleichsbeispiel 5a (mit 35% Toner eingefärbt) | 415 | 408 | 405 | 396 | 1,50 | 1,52 | 1,50 | 1,44 |
| Vergleichsbeispiel 5b (mit 59% Toner eingefärbt) | 210 | 207 | 208 | 209 | 1,48 | 1,86 | 1,94 | 1,80 |

**Tab. 2** zeigt, dass die die erfindungsgemäßen Messingpigmente gemäß Beispiel 1 enthaltenden Folienkonterapplikationen bei allen Druckvarianten einen höheren Glanz als die mit konventionellen Pigmenten gemäß Vergleichsbeispielen 4 und 5 pigmentierten Folienkonterapplikationen aufweisen.

Gegenüber Vergleichsbeispiel 4 weisen die Applikationen der erfindungsgemäßen Pigmente aus Beispiel 1 zudem eine höhere Farbdichte auf.

Der Glanz der Folienkonterapplikationen von Beispiel 1 war ebenfalls höher als bei den Vergleichsbeispielen 5a und 5b. Die höheren Farbdichten der Vergleichsbeispiele 5a und 5b suggerieren jedoch eine stärkere Farbigkeit dieser Applikationen als bei Beispiel 1. Dies war jedoch tatsächlich nicht der Fall.

Die visuelle Beurteilung des Spiegeleffektes der Folienkonterapplikationen ergab folgende Ergebnisse:

Beispiel 1: klarer, sehr guter Spiegel
Beispiel 4: Spiegel matt, trüb
Beispiel 5a: farbschwacher, silbriger Spiegel
Beispiel 5b: matter Spiegel

Zur weiteren optischen Charakterisierung wurden die Helligkeiten, die Buntheit und der Bunttonwinkel der pigmentierten Folienkonterapplikationen mit den in nachfolgender **Tab. 3** erfassten Versuchsergebnissen ermittelt. Es wurden Helligkeitsmessungen mit einem kommerziell erhältlichen Gerät der Fa. X-Rite (Lichtquelle. D65, 10° Normalbetrachter) in diffuser Meßgeometrie bei einem Beobachtungswinkel von 8° durchgeführt.

Hierbei wurden exemplarisch die Werte bei 60 l/cm vermessen.

Die in **Tab. 3** erfasste Buntheit **C**\* beschreibt die relative Sättigung im Verhältnis zum Referenzweiß, also im Vergleich zu einem bestimmten hellsten Punkt eines Farbraums. Der ebenfalls in **Tab. 3** erfasste Bunttonwinkel **h**\* ist der dem Farbton zugeordnete Farbwert, welcher auch als Buntton bezeichnet wird.

**Tab. 3 Optische Charakterisierung Pigmente II diffuse Meßgeometrie**

| Probe | L* | a* | b* | C* | H* | Glanz (60°) 60 l/cm |
|---|---|---|---|---|---|---|
| Beispiel 1 | 84,5 | 3,0 | 31,5 | 31,6 | 84,5 | 500 |
| Vergleichsbeispiel 4 | 78,8 | 3,6 | 27,9 | 28,2 | 82,7 | 243 |
| Vergleichsbeispiel 5a (mit 35% Toner eingefärbt) | 83,8 | 0,0 | 10,2 | 10,2 | 89,8 | 415 |
| Vergleichsbeispiel 5b (mit 59% Toner eingefärbt) | 77,2 | 0,2 | 27,4 | 27,4 | 89,5 | 210 |

Aus der **Tab. 3** ist zu entnehmen, dass die erfindungsgemäßen Messingpigmente gemäß Beispiel 1 farbintensiver waren als die der Vergleichsbeispiele 4 und 5. Diese Messungen entsprachen auch viel mehr dem visuellen Eindruck.

Dies bedeutet, dass das Goldbronzepigment aus Beispiel 1 aufgrund seiner geringen Pigmentdicke einen hohen Glanz und zusätzlich aufgrund seiner Eigenfarbe einen hohen Farbwert (Chroma) besaß. Weiterhin ist aus Tab. 3 ersichtlich, dass die optischen Eigenschaften der mit Toner eingefärbten Folienkonterapplikationen gemäß den Beispielen 5a und 5b mit der als Farbmittel eingesetzten Tonermenge korrelierten. So wiesen die mehr Farbmittel (Toner) enthaltenden Folienkonterapplikationen gemäß Beispiel 5b zwar ein höheres Chroma (Buntheit C*) aber geringere Helligkeiten L* und einen wesentlich verringerten Glanz (60°) als die weniger Farbmittel (Toner) enthaltenden Folienkonterapplikationen gemäß Beispiel 5a auf. Offenbar streuen die Farbpigmente zu sehr das Licht und vermindern so den metallischen Effekt. Diese Nachteile können mit den erfindungsgemäßen Metalleffektpigmenten überwunden werden.

Als weiteres Beurteilungskriterium wurde die Haftfestigkeit der pigmentierten Konterapplikationen mittels Tesa-Test (Spaltfestigkeit) ermittelt.
Hierzu wurde ein Klebestreifen fest und ohne Blasen auf die Oberfläche geklebt. Anschließend wurde dieser Klebestreifen wieder abgezogen, so dass der Untergrund nicht beschädigt wurde. Die Spaltfestigkeit wurde anhand eines Notensystems visuell von Note 1 (sehr gut) bis Note 5 (sehr schlecht) beurteilt. Eine schlechte Spaltfestigkeit spiegelt sich in einem entsprechend starken Ausriss aus dem Druck wieder.

Es wurde ermittelt, dass die erfindungsgemäßen Messingpigmente gemäß Beispiel 1 eine bessere Haftfestigkeit (Note 2) als die Goldbronzepigmente gemäß Vergleichsbeispiel 4 (Note 4) und die PVD-Aluminiumpigmente des Vergleichsbeispiels 5 (Note 3) aufweisen.

Zur Pigmentierung von wässrigen Anwendungen, beispielsweise Druckfarben, können die erfindungsgemäßen Messingpigmente mit einer die Pigmentoberfläche vollständig umschließenden Schutzschicht versehen werden. In nachfolgendem Beispiel 6 ist die Herstellung eines mit $SiO_2$-beschichtet Messingpigmentes beschrieben:

**Beispiel 6:**

55,1 g einer Paste mit Messingpigmenten nach Beispiel 1 (entspricht 38,6 g Messing) wurden in 375 ml Isopropanol dispergiert und auf Siedetemperatur gebracht. Es wurden 4,75 g Tetraethoxysilan zugegeben. Anschließend wurde über einen Zeitraum von 3 h eine Lösung von 4 g 25 %-igen $NH_3$ in 5 g Wasser hinzudosiert. Nach weiteren 3 h wurde auf Raumtemperatur abgekühlt und die Suspension über einen Büchnertrichter abgenutscht. Anschließend wurde das Produkt über Nacht in einem Vakuumtrockner bei 100°C getrocknet.

Die Pigmente wiesen sehr gute Ergebnisse im $H_2S$-Belastungstest auf. Weiterhin wurden die Pigmente in verschiedenen wässrigen Druckfarbensystemen eingearbeitet. Dabei wurden teilweise gar keine und teilweise nicht mehr als bei sonst üblichen passivierten Goldbronzepigmenten (Pigmente der Reihe Dorolan®, Fa. Eckart) zu beobachtenden Agglomerationsphänomenen bzw. Grünfärbungen erhalten.

Grünfärbungen bzw. Agglomerationen sind auf sich bildende Kupfer- bzw. Zinkionen zurückzuführen.

In einer Gesamtbetrachtung der Versuchsergebnisse ist festzustellen, dass die erfindungsgemäßen Pigmente eine bei konventionellen, durch Trockenvermahlung hergestellten Leafing Goldbronzepigmenten bisher nicht erreichte Pigmentcharakteristika, insbesondere hinsichtlich Dicke, Dickenverteilung und Deckkraft, aufweisen. Die mit erfindungsgemäßen Pigmenten pigmentierten Folienkonterapplikationen zeichnen sich durch attraktive koloristische Eigenschaften, insbesondere durch einen goldfarbenen Spiegeleffekt mit einer hohen Farbdichte aus, der bisher nicht mit Farbpigmente enthaltenen PVD-Aluminiumpigmenten erzielt werden konnte. Mit erfindungsgemäßen Pigmenten pigmentierte Folienkonterapplikationen besitzen eine hohe Haftfestigkeit. Zudem kann durch das hohe Deckvermögen der erfindungsgemäßen Pigmente deren Einsatzmenge im Anwendungsmedium verringert werden.

**Patentansprüche**

1. Plättchenförmige kupferhaltige Metalleffektpigmente, die einen Kupfergehalt von 60 bis 100 Gew.%, bezogen auf den gesamten Metallgehalt, aufweisen,
**dadurch gekennzeichnet,**
**dass** die Metallpigmente eine über Dickenauszählung mit Rasterelektronenmikroskopie (REM) ermittelte Dickenverteilung in der Darstellung als Summendurchgangsverteilung,

   a) mit einem $h_{50}$-Wert von 10 bis 50 nm,
   b) mit einem $h_{90}$-Wert von 20 bis 70 nm aufweisen,

wobei die plättchenförmigen kupferhaltigen Metalleffektpigmente durch Vermahlung eines kupferhaltigen Metallgrießes mit Schmiermittel hergestellt sind.

2. Plättchenförmige kupferhaltige Metalleffektpigmente nach Anspruch 1,
**dadurch gekennzeichnet,**
**dass** die Pigmente einen $h_{98}$-Wert von 21 bis unter 80 nm aufweisen.

3. Plättchenförmige kupferhaltige Metalleffektpigmente nach einem der vorstehenden Ansprüche,
**dadurch gekennzeichnet,**
**dass** die Pigmente einen $h_{10}$-Wert von 8 bis 25 nm aufweisen.

4. Plättchenförmige kupferhaltige Metalleffektpigmente nach einem der vorstehenden Ansprüche,
**dadurch gekennzeichnet,**
**dass** die Pigmente eine über Dickenauszählung mit Rasterelektronenmikroskopie (REM) ermittelte relative Breite der Dickenverteilung $\Delta h$, welche anhand der entsprechenden Summendurchgangskurve der relativen Häufigkeit nach der Formel $\Delta h = 100 \times (h_{90}-h_{10}) / h_{50}$ berechnet wird, von 30 % bis 90 %, aufweisen.

5. Plättchenförmige kupferhaltige Metalleffektpigmente nach einem der vorstehenden Ansprüche,
**dadurch gekennzeichnet,**
**dass** die Pigmente eine mittlere Größe $d_{50}$ im Bereich von 3 bis 50 $\mu$m aufweisen.

6. Plättchenförmige kupferhaltige Metalleffektpigmente nach einem der vorstehenden Ansprüche,
**dadurch gekennzeichnet,**
**dass** die Pigmente einen Formfaktor $d_{50}/h_{50}$ im Bereich von 150 bis 3.000 aufweisen.

7. Plättchenförmige kupferhaltige Metalleffektpigmente nach einem der vorherigen Ansprüche,
**dadurch gekennzeichnet,**
**dass** die Pigmente non-leafng Eigenschaften aufweisen.

8. Plättchenförmige kupferhaltige Metalleffektpigmente nach Anspruch 7,
**dadurch gekennzeichnet,**
**dass** die Oberfläche der Pigmente wenigstens teilweise mit einem Additiv bedeckt sind, wobei das Additiv als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind.

9. Plättchenförmige kupferhaltige Metalleffektpigmente nach einem der vorherigen Ansprüche,
**dadurch gekennzeichnet,**
**dass** die Pigmente mit einer passivierenden Inhibitor- und/oder Korrosionsschutzschicht belegt sind.

10. Verfahren zur Herstellung von plättchenförmigen kupferhaltigen Metalleffektpigmenten durch Vermahlung von Grieß nach einem der Ansprüche 1 bis 9,
umfassend folgenden Verfahrensschritt:

   Vermahlen eines kupferhaltigen Metallgrießes mit einer Korngrößenverteilung mit einem $d_{Grieß,50}$ von 1 bis 15 $\mu$m und einem $d_{Grieß,90}$ von 2 bis 27 $\mu$m und einem Kupfergehalt von 60 bis 100 Gew.-%, bezogen auf den gesamten Metallgrieß zu plättchenförmigen Metalleffektpigmenten unter Verwendung eines Mahlwerks in Gegenwart von Schmiermitteln und Mahlkörpern und optional Lösemittel,

wobei die so erhaltenen plättchenförmigen Metalleffektpigmente eine über Dickenauszählung mit Rasterelektronenmikroskopie (REM) ermittelte mittlere Dicke mit einem $h_{50}$-Wert von 10 bis 50 nm und einem $h_{90}$-Wert von 20 bis 70 nm aufweisen, wobei der kupferhaltige Metallgrieß ein Kupfer- oder Messinggrieß ist und einen Span $\Delta d_{Grieß} = (d_{Grieß} = (d_{Grieß,90}-d_{Grieß,10})/d_{Grieß,50}$ von 0,8 bis 1,7 aufweist.

**11.** Verfahren nach Anspruch 10,
**dadurch gekennzeichnet,**
**dass** der kupferhaltige Metallgrieß über einen Zeitraum von 10 bis 100 Stunden vermahlt wird.

**12.** Verfahren nach einem der Ansprüche 10 oder 11,
**dadurch gekennzeichnet,**
**dass** als Schmiermittel ein Additiv verwendet wird, wobei das Additiv als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind.

**13.** Verwendung von plättchenförmigen kupferhaltigen Metalleffektpigmenten nach einem der Ansprüche 1 bis 9 in Coatings, Anstrichen, Lacken, Druckfarben, Pulverlacken, Kunststoffen, Wertschriften- und Sicherheitsdrucken, Keramiken und kosmetischen Formulierungen.

**14.** Beschichtungszusammensetzung, vorzugsweise Druckfarbe, enthaltend plättchenförmige kupferhaltige Metalleffektpigmente nach einem der Ansprüche 1 bis 9.

**Claims**

**1.** Platelet-shaped, copper-containing metal effect pigments having a copper content of 60 to 100 % relative to the total metal content,
**characterised in that**
the metal pigments have a thickness distribution determined on the basis of thickness counting by scanning electron microscopy (SEM) represented as cumulative undersize distribution

     a) with an $h_{50}$ value of 10 to 50 nm,
     b) with an $h_{90}$ value of 20 to 70 nm,

and the platelet-shaped, copper-containing metal effect pigments are produced by milling a copper-containing metal powder with lubricant.

**2.** Platelet-shaped, copper-containing metal effect pigments as claimed in claim 1,
**characterised in that**
the pigments have an $h_{98}$ value of 21 to below 80 nm.

**3.** Platelet-shaped, copper-containing metal effect pigments as claimed in one of the preceding claims,
**characterised in that**
the pigments have an $h_{10}$ value of 8 to 25 nm.

**4.** Platelet-shaped, copper-containing metal effect pigments as claimed in one of the preceding claims,
**characterised in that**
the pigments have a relative span of the thickness distribution $\Delta h$ determined on the basis of thickness counting by scanning electron microscopy (SEM), calculated on the basis of the corresponding cumulative undersize curve of the relative frequency using the formula $\Delta h = 100 \times (h_{90}-h_{10})/h_{50}$, of 30 % to 90 %.

**5.** Platelet-shaped, copper-containing metal effect pigments as claimed in one of the preceding claims,
**characterised in that**
the pigments have an average size $d_{50}$ in the range of 3 to 50 $\mu$m.

**6.** Platelet-shaped, copper-containing metal effect pigments as claimed in one of the preceding claims,
**characterised in that**
the pigments have a form factor $d_{50}/h_{50}$ in the range of 150 to 3,000.

7. Platelet-shaped, copper-containing metal effect pigments as claimed in one of the preceding claims, **characterised in that** the pigments have non-leafing properties.

8. Platelet-shaped, copper-containing metal effect pigments as claimed in claim 7, **characterised in that** the surface of the pigments is at least partially coated with an additive, and the additive comprises at least one carboxylic acid with at least 4 carbon atoms as well as at least one polyglycol ether as structural units, and the carboxylic acid and the polyglycol ether are covalently bonded to one another.

9. Platelet-shaped, copper-containing metal effect pigments as claimed in one of the preceding claims, **characterised in that** the pigments are coated with a passivating inhibitor layer and/or corrosion inhibiting layer.

10. Method of producing platelet-shaped, copper-containing metal effect pigments by milling powder as claimed in one of claims 1 to 9, comprising the following method step:

milling a copper-containing powder with a particle size distribution with a $d_{powder.50}$ of 1 to 15 $\mu$m and a $d_{powder.90}$ of 2 to 27 $\mu$m and a copper content of 60 to 100 % by weight relative to the total metal powder to obtain platelet-shaped metal effect pigments using a mill in the presence of lubricants and grinding media and optionally solvents, the platelet-shaped metal effect pigments obtained in this manner having an average thickness determined on the basis of thickness counting by scanning electron microscopy (SEM) with an $h_{50}$ value of 10 to 50 nm and an $h_{90}$ value of 20 to 70 nm, and the copper-containing metal powder is a copper powder or brass powder and has a span $\Delta d_{powder} = (d_{powder.90} - d_{powder.10})/d_{powder.50}$ of 0.8 to 1.7.

11. Method as claimed in claim 10, **characterised in that** the copper-containing metal powder is milled for a period of 10 to 100 hours.

12. Method as claimed in one of claims 10 or 11, **characterised in that** an additive is used as a lubricant, and the additive comprises at least one carboxylic acid with at least 4 carbon atoms as well as at least one polyglycol ether as structural units, and the carboxylic acid and the polyglycol ether are covalently bonded to one another.

13. Use of platelet-shaped, copper-containing metal effect pigments as claimed in one of claims 1 to 9 in coatings, paints, varnishes, printing inks, powder coatings, plastics, security and counterfeitproof inks, ceramics and cosmetic formulations.

14. Coating composition, preferably a printing ink, containing platelet-shaped, copper-containing metal-effect pigments as claimed in one of claims 1 to 9.

## Revendications

1. Pigments métalliques à effets, lamellaires, contenant du cuivre, qui présentent une teneur en cuivre de 60 à 100 % en poids par rapport à la totalité du métal contenu, **caractérisés en ce que** les pigments métalliques présentent une distribution des épaisseurs, déterminée par le décompte des épaisseurs au microscope électronique à balayage (MEB), dans une représentation en tant que distribution des fréquences cumulées, ayant

a) une valeur $h_{50}$ de 10 à 50 nm,
b) une valeur $h_{90}$ de 20 à 70 nm,
les pigments métalliques à effets, lamellaires, contenant du cuivre, étant fabriqués par broyage, avec un lubrifiant, d'une grenaille métallique contenant du cuivre.

2. Pigments métalliques à effets, lamellaires, contenant du cuivre, selon la revendication 1, **caractérisés en ce que** les pigments présentent une valeur $h_{98}$ de 21 à 80 nm.

3. Pigments métalliques à effets, lamellaires, contenant du cuivre, selon l'une des revendications précédentes, **caractérisés en ce que** les pigments présentent une valeur $h_{10}$ de 8 à 25 nm.

4. Pigments métalliques à effets, lamellaires, contenant du cuivre, selon l'une des revendications précédentes, **caractérisés en ce que** les pigments présentent une largeur relative, déterminée par le décompte des épaisseurs au microscope électronique à balayage (MEB), de la distribution des épaisseurs $\Delta h$, laquelle est calculée à l'aide de la courbe de passage cumulée correspondante de la fréquence relative selon la formule $\Delta h = 100 \times (h_{90}-h_{10})/h_{50}$, de 30 à 90 %.

5. Pigments métalliques à effets, lamellaires, contenant du cuivre, selon l'une des revendications précédentes, **caractérisés en ce que** les pigments présentent une granulométrie moyenne $d_{50}$ comprise dans la plage de 3 à 50 $\mu$m.

6. Pigments métalliques à effets, lamellaires, contenant du cuivre, selon l'une des revendications précédentes, **caractérisés en ce que** les pigments présentent un facteur de forme $d_{50}/h_{50}$ compris dans la plage de 150 à 3000.

7. Pigments métalliques à effets, lamellaires, contenant du cuivre, selon l'une des revendications précédentes, **caractérisés en ce que** les pigments présentent des caractéristiques non pelliculantes.

8. Pigments métalliques à effets, lamellaires, contenant du cuivre, selon la revendication 7, **caractérisés en ce que** la surface des pigments est au moins partiellement recouverte d'un additif, l'additif comprenant en tant que motifs structuraux au moins un acide carboxylique ayant au moins 4 atomes de carbone, ainsi qu'au moins un polyglycoléther, l'acide carboxylique et le polyglycoéther étant liés l'un à l'autre par une liaison covalente.

9. Pigments métalliques à effets, lamellaires, contenant du cuivre, selon l'une des revendications précédentes, **caractérisés en ce que** les pigments sont revêtus d'une couche passivante d'inhibiteur et/ou de protection anticorrosion.

10. Procédé de fabrication de pigments métalliques à effets, lamellaires, contenant du cuivre, par broyage d'une grenaille selon l'une des revendications 1 à 9, comprenant l'étape suivante :

   broyage d'une grenaille métallique contenant du cuivre, ayant une distribution granulométrique correspondant à un $d_{grenaille,50}$ de 1 à 15 $\mu$m et un $d_{grenaille,90}$ de 2 à 27 $\mu$m, et une teneur en cuivre de 60 à 100 % en poids par rapport à la totalité de la grenaille métallique des pigments métalliques à effets, lamellaires, par utilisation d'un broyeur en présence de lubrifiants et d'éléments broyeurs et en option d'un solvant,
   les pigments métalliques à effets, lamellaires, ainsi obtenus présentant une épaisseur moyenne, déterminée par le décompte des épaisseurs au microscope électronique à balayage (MEB), ayant une valeur $h_{50}$ de 10 à 50 nm et une valeur $h_{90}$ de 20 à 70 nm, la grenaille métallique contenant du cuivre étant une grenaille de cuivre ou de laiton, et présentant une plage $\Delta d_{grenaille} = (d_{grenaille,90}-d_{grenaille,10})/d_{grenaille,50}$, de 0,8 à 1,7.

11. Procédé selon la revendication 10, **caractérisé en ce que** la grenaille métallique contenant du cuivre est broyée pendant un laps de temps de 10 à 100 heures.

12. Procédé selon l'une des revendications 10 ou 11, **caractérisé en ce qu'**on utilise en tant que lubrifiant un additif, l'additif comprenant en tant que motifs structuraux au moins un acide carboxylique ayant au moins 4 atomes de carbone, ainsi qu'au moins un polyglycoléther, l'acide carboxylique et le polyglycoléther étant liés l'un à l'autre par une liaison covalente.

13. Utilisation de pigments métalliques à effets, lamellaires, contenant du cuivre, selon l'une des revendications 1 à 9, dans des revêtements, des peintures, des vernis, des encres d'imprimerie, des peintures en poudre, des matières plastiques, des titres et documents imprimés de sécurité, des céramiques et des formulations cosmétiques.

14. Composition de revêtement, de préférence encre d'imprimerie, contenant des pigments métalliques à effets, lamellaires, contenant du cuivre, selon l'une des revendications 1 à 9.

Abb. 1: Dickenverteilung Beispiele

Teilchendicke [nm]

Kumulierte Häufigkeit

- ■ Beispiel 1
- —— Fit Beispiel 1
- ◆ Beispiel 2
- – · – Fit Beispiel 2
- □ Vergleichsbeispiel 4
- – – Fit Vergleichsbeispiel 4
- △ Vergleichsbeispiel 5
- ······ Fit Vergleichsbeispiel 5

**EP 2 285 913 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1529084 B1
- DE 2007717 A
- JP 63000406 A
- JP 2002327201 A
- US 2002891 A
- US 3995815 A
- US 4172720 A
- US 4884754 A
- US 20020050186 A1

- WO 2008077612 A2
- WO 2004026972 A
- US 6398861 B1
- DE 10315775 A1
- EP 1304210 A1
- DE 202004005921 U1
- WO 0236695 A
- WO 0236697 A